# EUROPEAN PATENT SPECIFICATION

(11) **EP 3 746 179 B1**
(45) Date of publication and mention of the grant of the patent: **13.05.2026**
(21) Application number: 19747382.0
(22) Date of filing: 04.02.2019
(51) Int. Cl.: A61F 2/02, A61L 27/38, A61L 27/18, A61L 27/54, A61L 27/56, A61P 3/10, C12N 5/077

(54) **METHODS AND COMPOSITIONS FOR TREATING AND PREVENTING DIABETES**
VERFAHREN UND ZUSAMMENSETZUNGEN ZUR BEHANDLUNG UND VORBEUGUNG VON DIABETES
MÉTHODES ET COMPOSITIONS POUR LE TRAITEMENT ET LA PRÉVENTION DU DIABÈTE

(30) Priority: 04.02.2018 US 201862626098 P
(43) Date of publication of application: 09.12.2020
(73) Proprietor: Technion Research & Development Foundation Limited, Haifa 3200004 (IL); B.G. Negev Technologies and Applications Ltd., at Ben-Gurion University, 84105 Beer-Sheva (IL)
(72) Inventor: LEVENBERG, Shulamit, 2018600 Moreshet (IL); KARNIELI, Eddy, 3608240 Kiriat Tivon (IL); LEWIS, Eli Chaim, 8481932 Beer Sheva (IL); BECKERMAN, Margarita, 2303803 Migdal Haemek (IL); HAREL, Chava, 3475041 Haifa (IL)
(74) Representative: Boult Wade Tennant LLP
(86) International application number: PCT/IL2019/050134
(87) International publication number: WO 2019/150377

(56) References cited:
- EP-A1- 3 272 877
- WO-A1-2008/111064
- US-A1- 2008 038 236
- US-A1- 2008 102 054
- ATKINSON B. J. ET AL: "Moderate GLUT4 Overexpression Improves Insulin Sensitivity and Fasting Triglyceridemia in High-Fat Diet-Fed Transgenic Mice", vol. 62, no. 7, 1 July 2013 (2013-07-01), US, pages 2249 - 2258, XP055844970, ISSN: 0012-1797, Retrieved from the Internet <URL:https://www.ncbi.nlm.nih.gov/pmc/articles/PMC3712063/pdf/2249.pdf> DOI: 10.2337/db12-1146
- OTAEGUI PEDRO J. ET AL: "Glucose-Regulated Glucose Uptake by Transplanted Muscle Cells Expressing Glucokinase Counteracts Diabetic Hyperglycemia", HUMAN GENE THERAPY, vol. 13, no. 18, 10 December 2002 (2002-12-10), GB, pages 2125 - 2133, XP055844958, ISSN: 1043-0342, DOI: 10.1089/104303402320987824
- MICHAEL L F ET AL: "Restoration of insulin-sensitive glucose transporter (GLUT4) gene expression in muscle cells by the transcriptional coactivator PGC-1", 27 March 2001, PROCEEDINGS OF THE NATIONAL ACADEMY OF SCIENCES, NATIONAL ACADEMY OF SCIENCES, US, PAGE(S) 3820 - 3825, ISSN: 0027-8424, XP002225764
- MAUNEY, JOSHUA R. ET AL.: "Engineering adipose-like tissue in vitro and in vivo utilizing human bone marrow and adipose-derived mesenchymal stem cells with silk fibroin 3D scaffolds", BIOMATERIALS, vol. 28.35, 31 December 2007 (2007-12-31), pages 5280 - 5290, XP022290568

## Description

### FIELD OF INVENTION

The present invention is in the field of biomedical engineering.

### BACKGROUND OF THE INVENTION

Diabetes mellitus (DM) is a disease that occurs in all populations and age groups and affects more than 10% of the Western world. It is the sixth leading cause of death in the United States, affecting between 6% and 7% of the US population equating to about 16 million people. The two most common general categories of this disease are termed type 1 diabetes (DM1) and type 2 diabetes (DM2). The number of people with diabetes is expected to reach 300 million in 2030, of which 90% will be DM2. Obesity is a major environmental factor contributing to the increasing incidence of DM2. Modern lifestyle, high-fat diet and lack of exercise were shown to trigger the development of DM2 in overweight patients with impaired glucose tolerance, while increased levels of markers and mediators of inflammation and oxidative stress components correlated with impaired insulin action (insulin resistance).

DM2 is a heterogeneous, polygenic disorder characterized by defects in insulin action in tissues (insulin resistance) and/or defects in pancreatic insulin secretion (beta cell dysfunction), which eventually results in loss of pancreatic insulin-secreting (beta) cells. The associated complications of diabetes are cardiovascular disease, peripheral vascular disease, stroke, diabetic neuropathy, diabetic nephropathy and diabetic retinopathy. These result in increasing disability, reduced life expectancy and enormous health costs. The development of these diabetes-related complications can be significantly reduced, partially prevented and retarded with control of blood glucose levels as close to normal as possible. However, in spite of the current knowledge and new treatment protocols, many patients currently do not reach the desired treatment goals. DM2 is a progressive and complex disorder that is difficult to treat effectively in the long term. The treatment begins with a well-balanced diet combined with exercise. Unfortunately, the majority of patients are unable to achieve or sustain near normo-glycemia without oral antidiabetic agents; a sizeable proportion of patients will eventually require oral and/or injectable hypoglycemic drugs and/or insulin therapy to maintain long-term glycemic control. The frequent need for escalating therapy reflects progressive loss of islet beta-cell function, usually in the presence of obesity-related insulin resistance.

Insulin resistance is a key component in the pathogenesis of DM2. It is a state of resistance to the action of insulin in its target tissues, i.e., impaired insulin stimulation of glucose transport in adipose tissue and skeletal muscle, and reduced inhibition of glucose production and release in the liver. One of the earliest defects detected in DM2 is reduction of cellular content and an impaired function of the insulin-responsive glucose transporter type 4, a member of the glucose transport proteins (GLUTs) mediating glucose uptake in eukaryotic cells. GLUT4 is the main glucose transporter regulating glucose entry from the blood into adipose and muscle tissues upon insulin stimulation.

Fussenegger and Xie (EP Patent No. 3272877) describe methods for producing β-cell-mimetic cells as well as methods of using the β-cell-mimetic cells as a medicament and methods of using β-cell-mimetic cells for the prevention, delay of progression or treatment of a metabolic disease in a subject.

Levenberg and Francis (International Patent Application No. PCT/IL2008/000337, published as WO2008111064) describe a heterogeneous population of cells seeded on a surface of a scaffold, wherein the heterogeneous population of cells includes at least one pancreatic islet, endothelial cells and fibroblast cells, as well as methods of generating same and uses thereof.

Atkinson et al., 2013 describe that moderate GLUT4 overexpression improves insulin sensitivity and fasting triglyceridemia in high-fat diet-fed transgenic mice.

Otaegui et al., 2002 describe that glucose-regulated glucose uptake by transplanted muscle cells expressing glucokinase counteracts diabetic hyperglycemia.

Michael et al., 2001 describe the restoration of insulin-sensitive glucose transporter (GLUT4) gene expression in muscle cells by the transcriptional coactivator PGC-1.

### SUMMARY OF THE INVENTION

According to the first aspect, there is provided a scaffold-cell construct comprising: (a) a porous scaffold comprising at least one biocompatible polymer comprising a combination of poly-1-lactic acid (PLLA) and polylactic glycolic acid (PLGA), or collagen; and (b) a cell population deposited on or in the scaffold comprising recombinant myoblasts: (i) comprising an exogenous polynucleotide encoding glucose transporter type 4 (GLUT4); (ii) over-expressing the GLUT4; and (iii) having increased GLUT4 activity, compared to control wild type myoblast cells, and wherein the scaffold-cell construct is transplantable.

According to another aspect, there is provided a composition comprising the scaffold-cell construct of the invention.

According to another aspect, there is provided a method of making a scaffold-cell construct for restoring glucose homeostatic levels in a diabetic subject in need thereof, the method comprising contacting recombinant myoblasts: (i) comprising an exogenous polynucleotide encoding GLUT4; (ii) over-expressing the GLUT4; and (iii) having increased GLUT4 activity, compared to control wild type myoblast cells, with a scaffold comprising at least one biocompatible polymer comprising a combination of PLLA and PLGA or collagen, and wherein the scaffold-cell construct is transplantable.

In some embodiments, the scaffold-cell construct is characterized by having greater glucose uptake rates compared to the control wild type myoblast cells.

In some embodiments, the PLLA and the PLGA are in a ratio of 3:1 - 1:3 w/w ratio.

In some embodiments, the biocompatible polymer comprises interconnected pores, wherein at least 80% of the pores have a diameter of between 200 and 600 microns.

In some embodiments, the scaffold-cell construct is for use in restoring glucose homeostasis levels in a subject in need thereof, wherein: (a) the glucose levels are: (i) less than or equal to 100 mg/dL at fasting; and (ii) lower than 140 mg/dL postprandial; or (b) the glucose levels are: (i) less than or equal to 120 mg/dL at fasting; and (ii) lower than 160 mg/dL postprandial.

In some embodiments, the scaffold-cell construct is for use in the treatment or prevention of diabetes mellitus or metabolic syndrome in a subject in need thereof, preferably wherein said metabolic syndrome is selected from: obesity, pre-diabetes and insulin resistance or related to insulin resistance.

In some embodiments, the method further comprises culturing the recombinant myoblasts on or in the scaffold for at least 7 days.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Further embodiments and the full scope of applicability of the present invention will become apparent from the detailed description given hereinafter. However, it should be understood that the detailed description and specific examples, while indicating preferred embodiments of the invention, are given by way of illustration only.

### BRIEF DESCRITPION OF THE DRAWINGS

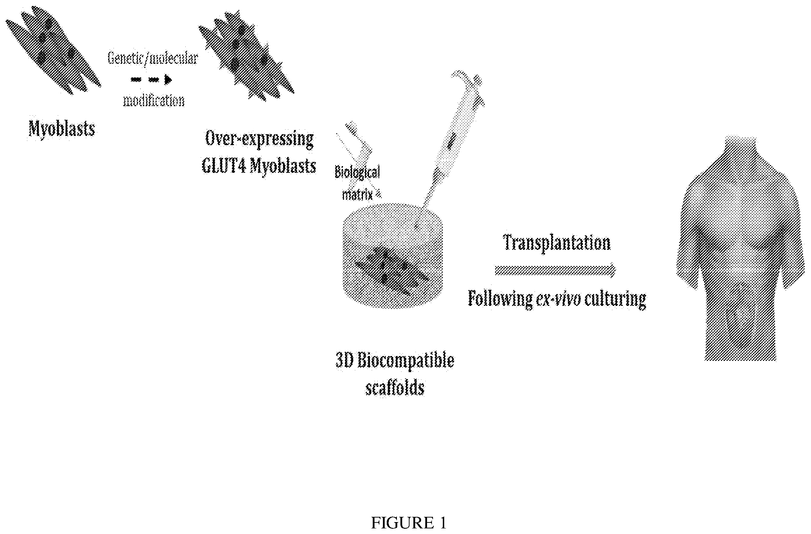

**Fig. 1** is a schematic presentation of a non-limiting example of the disclosed invention, by which, myoblasts are genetically-/molecularly-modified to over express GLUT4. The over-expressing GLUT4 myoblasts are cultured *ex-vivo* on 3D biocompatible scaffolds and transplanted thereafter.

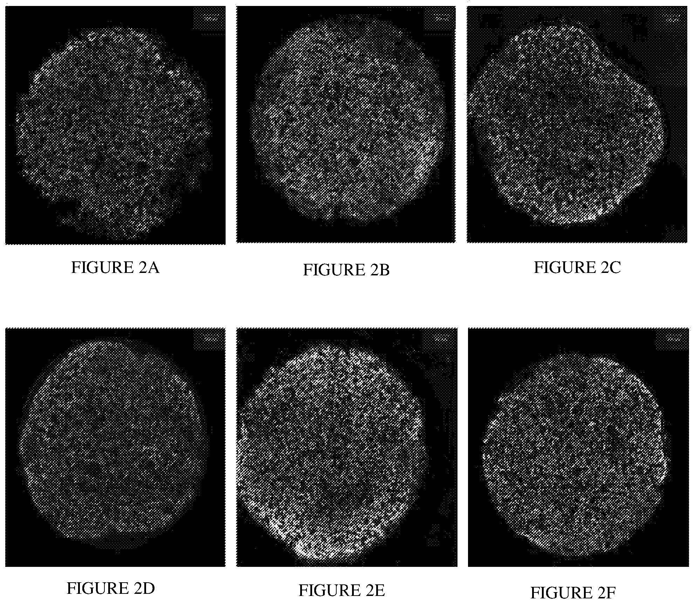

**Figs. 2A-2F** are representative immunofluorescent micrographs of whole poly-1-lactic acid (PLLA)/polylactic glycolic acid (PLGA) scaffolds seeded with 0.5 × 10⁶ L6 cells, either wild-type (L6WT; **2A, 2C and 2E**) or GLUT4 over-expressing cells (L6GLUT4; **2B, 2D and 2F**), and grown for 1 week *in-vitro.* Scaffolds were stained for different muscular markers, e.g., desmin (**2A-2B**), myosin heavy chain (MYH; **2C-2D**), and myogenin (MYOG; **2E-2F**) (all stained in red), all of which were shown to colocalize with the GLUT4 transporter (green; **2A-2F**). Scale bar = 500 µm.

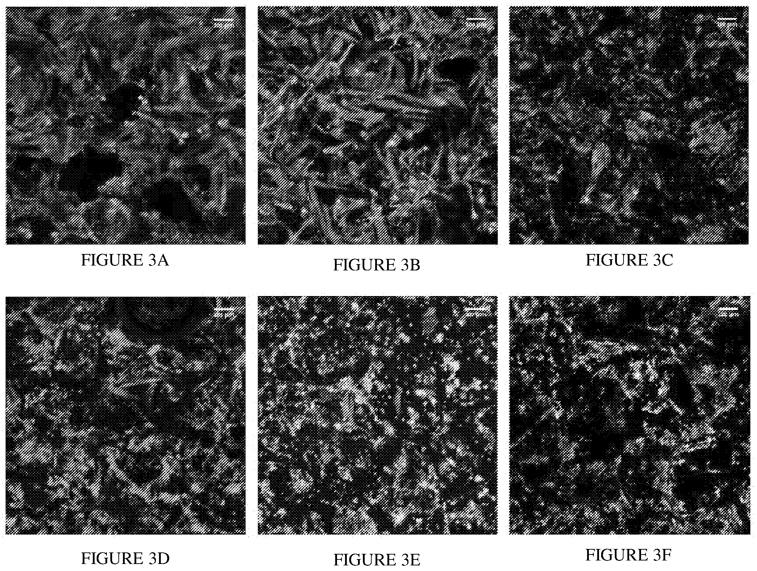

**Figs. 3A-3F** are representative immunofluorescent micrographs of whole poly-1-lactic acid (PLLA)/polylactic glycolic acid (PLGA) scaffolds seeded with 0.5 × 10⁶ L6 cells, either wild-type (L6WT; **3A, 3C and 3E**) or GLUT4 over-expressing cells (L6GLUT4; **3B, 3D and 3F**), and grown for 1 week *in-vitro.* Scaffolds were stained for different muscular markers, e.g., desmin (**3A-3B**), myosin heavy chain (MYH; **3C-3D**), and myogenin (MYOG; **3E-3F**) (all stained in red), all of which were shown to colocalize with the GLUT4 transporter (green; **3A-3F**). Scale bar = 100 µm.

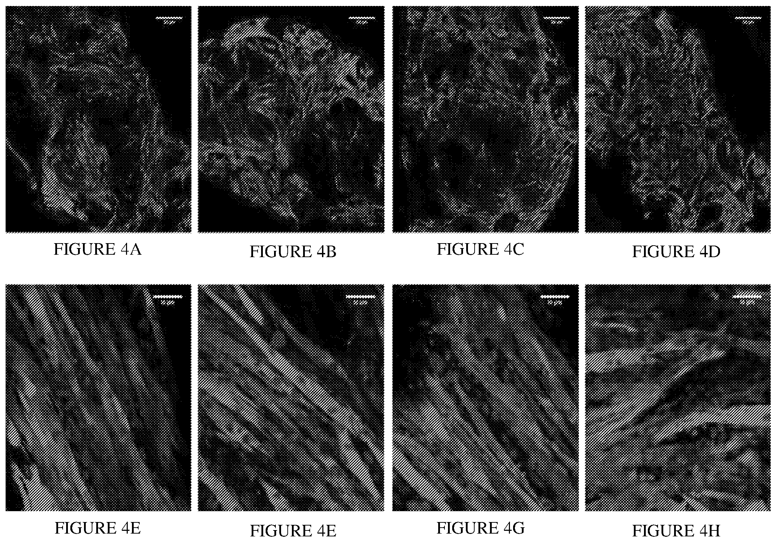

**Figs. 4A-4H** are representative immunofluorescent micrographs of whole poly-1-lactic acid (PLLA)/polylactic glycolic acid (PLGA) scaffolds seeded with 0.5 × 10⁶ L6 cells, either wild-type (L6WT; **4A, 4B, 4E and 4F**) or GLUT4 over-expressing cells (L6GLUT4; **4C, 4D, 4G and 4H**), and grown for 2 (**4A, 4C, 4E and 4G**) and 3 (**4B, 4D, 4F and 4H**) weeks *in-vitro.* Scaffolds were shown to comprise elongated, defined and aligned muscle fibers (stained in red using the muscular marker desmin). Round cell nuclei were also visualized (stained in blue using DAPI). Upper panel: scale bar = 500 µm. Lower panel: scale bar = 50 µm. (**4E-4H**) are representative enlargements of selected areas from (**4A-4D**), respectively.

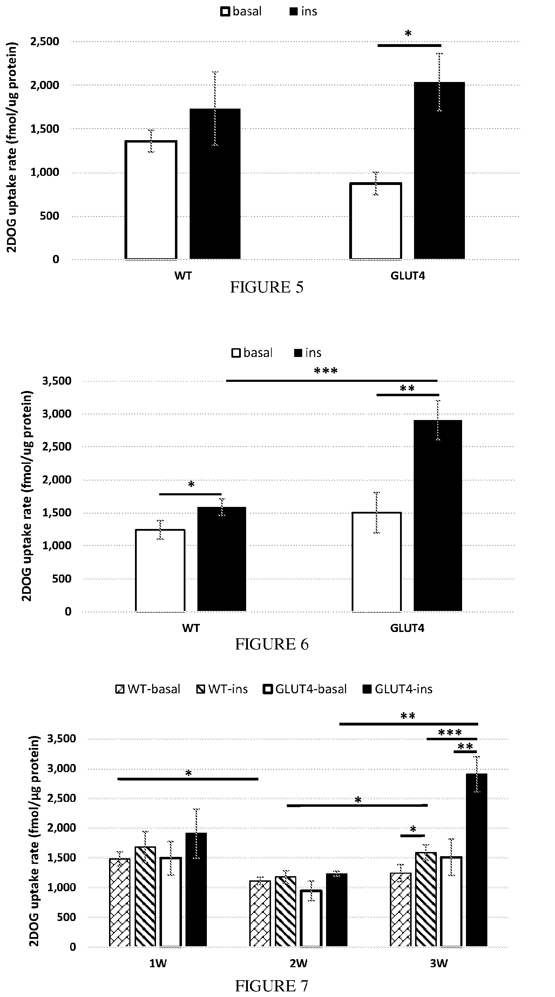

**Fig. 5** is vertical bar charts of 2-[³H]-deoxyglucose (2DOG) uptake rates in 0.25 × 10⁶ L6 cells, either wild-type (WT) or GLUT4 over-expressing cells (GLUT4) grown on poly-1-lactic acid (PLLA)/polylactic glycolic acid (PLGA) scaffolds for 3 weeks *in-vitro* (*p<0.05).
**Fig. 6** is vertical bar charts of 2-[³H]-deoxyglucose (2DOG) uptake rates in 0.5 × 10⁶ L6 cells, either wild-type (WT) or GLUT4 over-expressing cells (GLUT4) grown on poly-1-lactic acid (PLLA)/polylactic glycolic acid (PLGA) scaffolds for 3 weeks *in-vitro* (*p<0.05; **p,0.01; ***p<0.005).
**Fig. 7** is vertical bar charts of 2-[³H]-deoxyglucose (2DOG) uptake rates in 0.5 × 10⁶ L6 cells, either wild-type (WT) or GLUT4 over-expressing cells (GLUT4) grown on poly-l-lactic acid (PLLA)/polylactic glycolic acid (PLGA) scaffolds for 1, 2 or 3 weeks *in-vitro* (1W, 2W and 3W, respectively). After each culturing time, cells were serum-starved, followed by incubation in the absence/presence of insulin (-basal/-ins, respectively). 2DOG uptake was then measured (*p<0.05; **p,0.01; ***p<0.005).

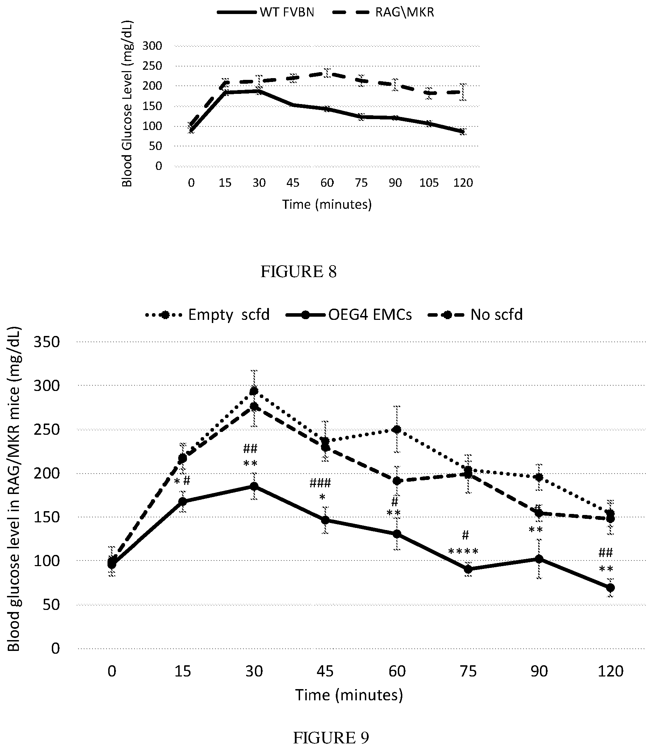

**Fig. 8** is a graph demonstrating glycemic activity of immuno-deficient mice with no mature B and T cells (i.e., RAG), which bear a genetically defective insulin receptor (i.e., MKR) thus are insulin resistant and type 2 diabetes mellitus-afflicted, following glucose tolerance test (GTT). WT-FVBN mice, which are the background strain for RAG/MKR, were used as reference.
**Fig. 9** is a graph of a glucose tolerance test (GTT), which was performed in RAG/MKR mice implanted with poly-l-lactic acid (PLLA)/polylactic glycolic acid (PLGA) scaffolds seeded with L6GLUT4 cells (OEG4 EMCs), mice implanted with empty scaffolds (Empty scfd), and non-implanted control mice (no scfd). Difference between no scfd and OEG4 * p<0.05; ** p<0.01, **** p<0.0001; Difference between Empty and OEG4 # p<0.05; ## p<0.01, ### p<0.001.

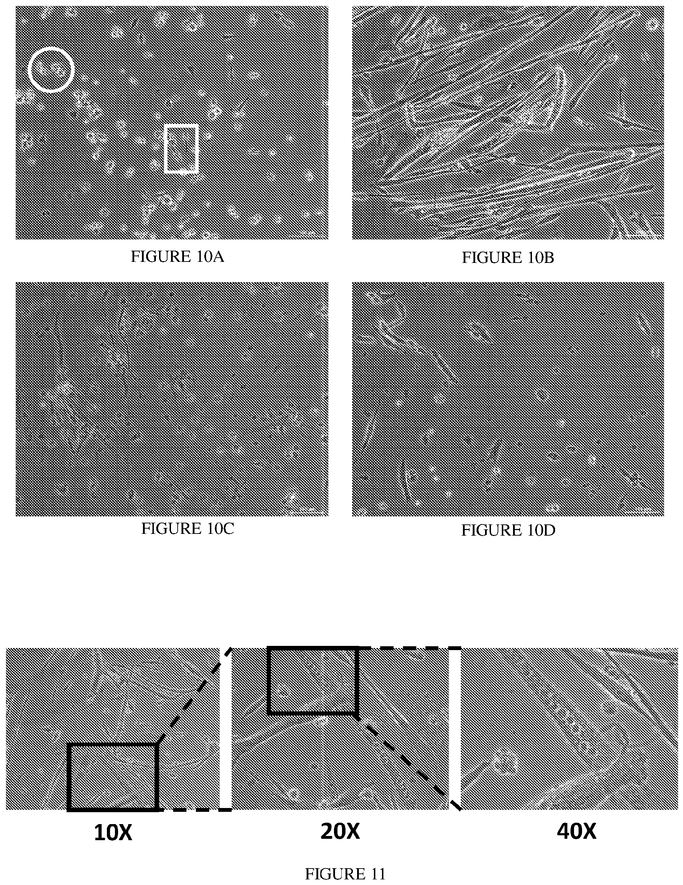

**Figs. 10A-10D** are micrographs showing differentiation of C57-SC with different mediums, scale bar: 100 µm. **(10A)** BIO-AMF-2; **(10B)** DMEM 5% HS; **(10C)** DMEM 2% FBS; and **(10D)** SKMSM 2% FBS.
**Fig. 11** is micrographs of enlarged images of myotubes formed out of differentiated C57-SC.

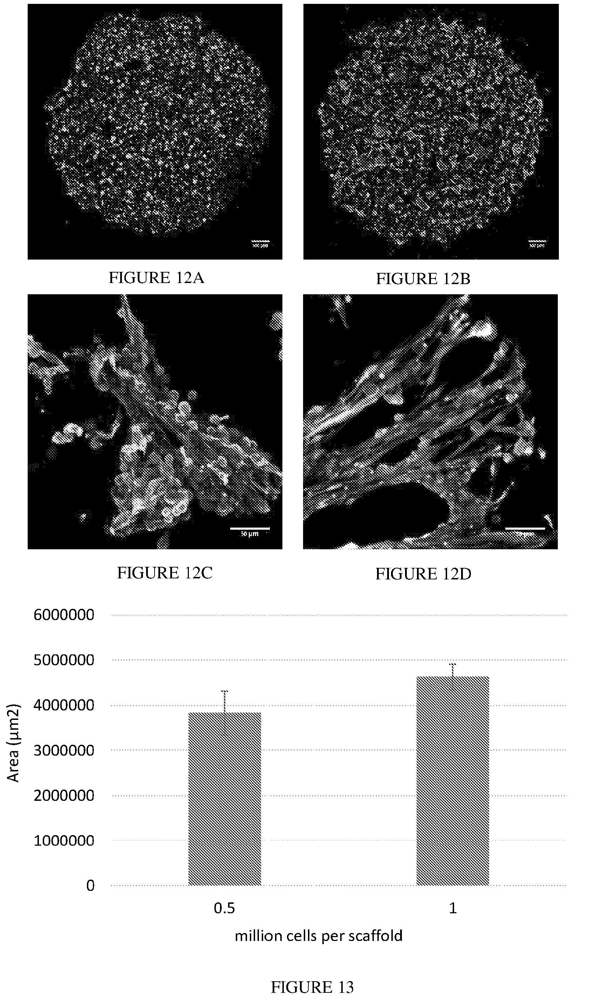

**Figs. 12A-12D** are immunofluorescent micrographs of whole scaffold stain of PLLA\PLGA scaffolds seeded with 0.5 × 10⁶ **(12A)** or 1 × 10⁶ **(12B)** C57-SC cells grown 3 weeks *in-vitro,* stained for desmin (green), MYOG (magenta) and cell nuclei with DAPI (blue). **(12C and 12D)** are enlarged areas of **(12A and 12B),** respectively. Scale bar: 500 µm **(12A and 12B)**; 50 µm **(12C and 12D).**
**Fig. 13** is a vertical bar graph showing the quantification of desmin signal for whole scaffold stain of PLLA\PLGA scaffolds seeded with 0.5 × 10⁶ and 1 × 10⁶ C57-SC cells grown 3 weeks *in-vitro.*

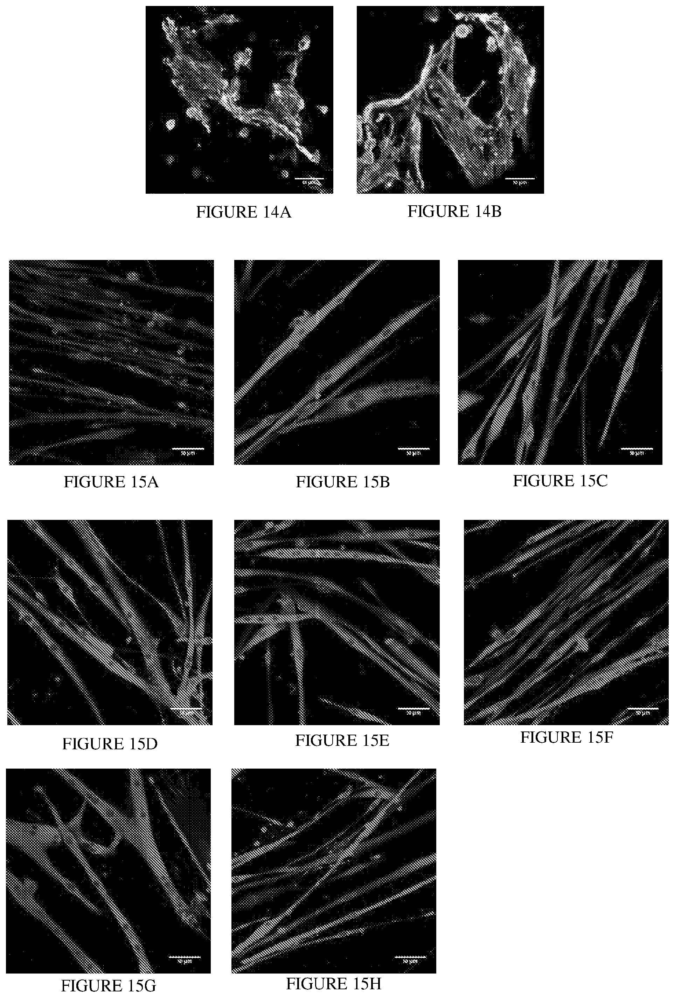

**Figs. 14A-14B** are immunofluorescent micrographs showing representative enlarged (20×) images of whole PLLA\PLGA scaffolds seeded with 0.5 × 10⁶ **(14A)** or 1 × 10⁶ **(14B)** C57-SC cells grown 3 weeks *in-vitro,* stained for desmin (green), MYOG (magenta) and cell nuclei with DAPI (blue), scale bar: 50 µm.
**Figs. 15A-15H** are immunofluorescent micrographs showing staining for GLUT4 transporter (red) and cell nuclei with DAPI (blue) in C57-SC myotubes grown in 2D culture for 1 week, scale bar: 50 µm. Wild type **(15A);** Clones: PB A12 **(15B),** PB A34 **(15C),** PB A56 **(15D),** PB B12 **(15E),** RN A12 **(15F),** RN A34 **(15G),** RN A56 **(15H).**

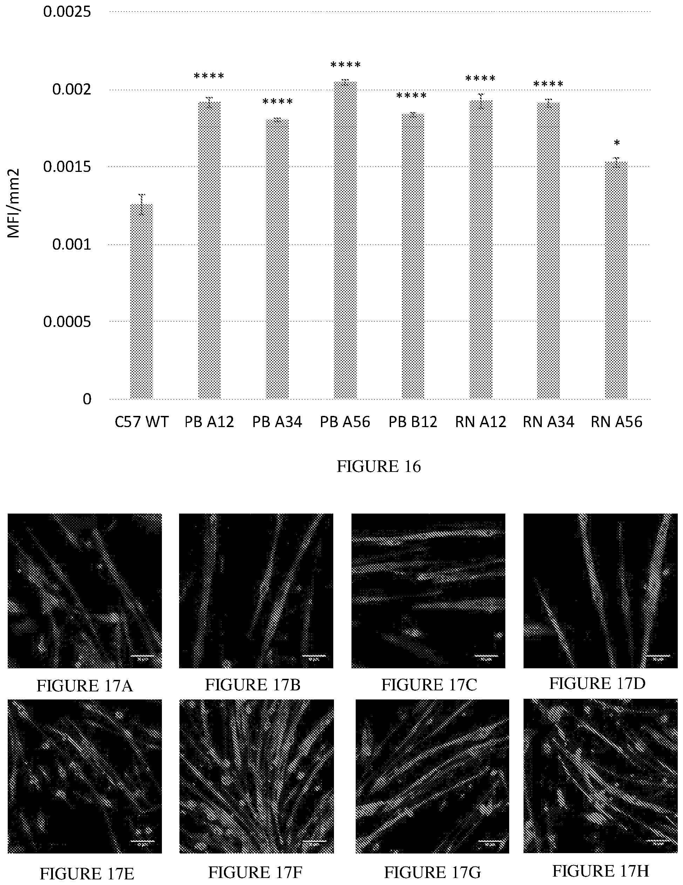

**Fig. 16** is a vertical bar graph showing GLUT4 signal intensity in C57-SC myotubes grown in 2D culture for 1 week. * p<0.05, **** p<0.0001.
**Figs. 17A-17H** are immunofluorescent micrographs showing staining for GLUT4 transporter (red) and cell nuclei with DAPI (blue) in C57-SC myotubes grown in 2D culture for 1 week, scale bar: 50µm. Wild type **(17A);** Clones: PB B34 **(17B),** PB B56 **(17C),** PB C12 **(17D),** RN B12 **(17E),** RN B34 **(17F),** RN B56 **(17G),** RN C12 **(17H).**

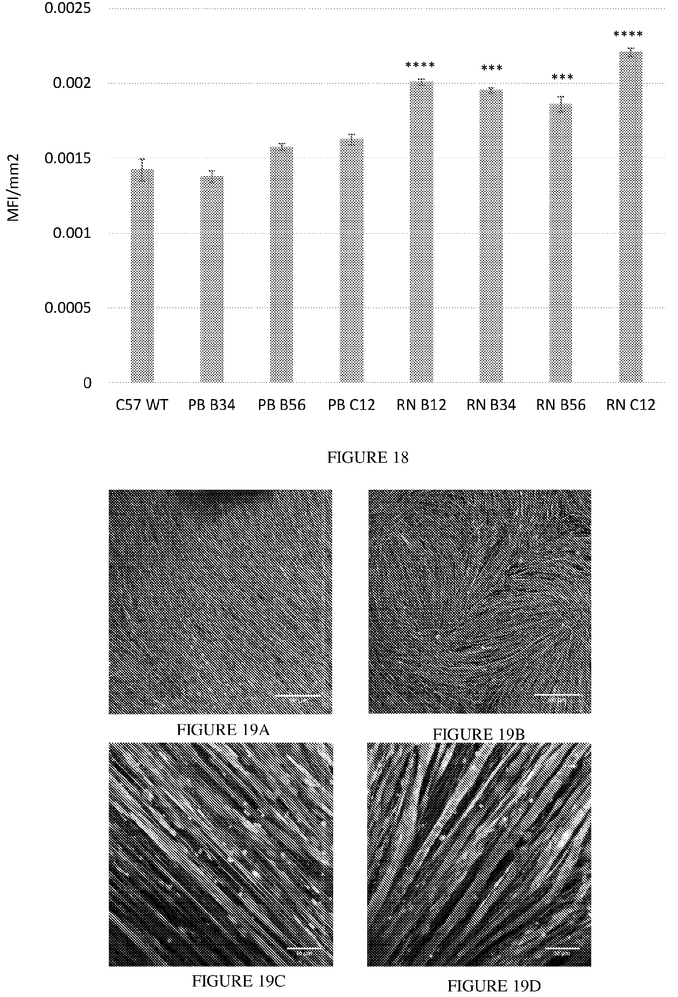

**Fig. 18** is a vertical bar graph showing GLUT4 signal intensity in C57-SC myotubes grown in 2D culture for 1 week. *** p<0.001, **** p<0.0001.
**Figs. 19A-19H** are immunofluorescent micrographs showing C57-SC myotubes grown in 2D culture for 1 week, stained for different myogenic markers and cell nuclei with DAPI. **(19A-19D)** are images of the following stains: desmin (green), MYOG (magenta) and cell nuclei (blue). **(19E-19H)** are images of the following stains: MYH (green), MYOG (magenta) and cell nuclei (blue). **(19C and 19D)** are enlarged areas of **(19A and 19B),** respectively. **(19G and 19H)** are enlarged areas of **(19E and 19F),** respectively. Scale bar: 250 µm **(19A, 19B, 19E and 19F);** 50 µm **(19C, 19D, 19G and 19H).** Clones: PB A12 **(19A, 19C, 19E, and 19G);** RN B12 **(19B, 19D, 19F and 19H).**

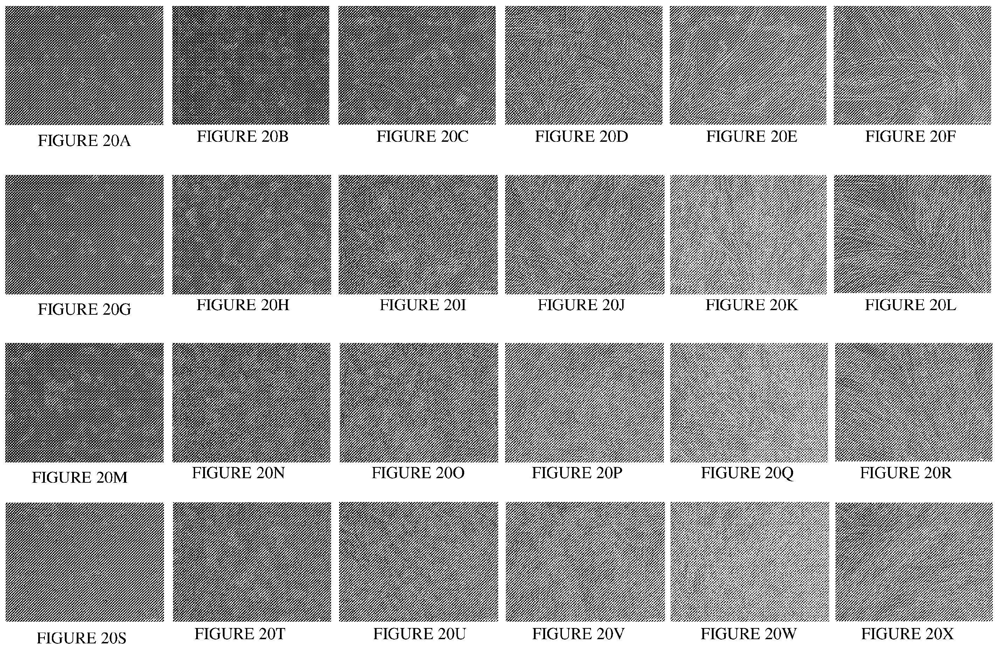

**Figs. 20A-20X** are micrographs of 2D myotube formation of WT C57-SC. Scale bar: 100 µm. Cell number: **(20A-20F)** 50,000 cells; **(20G-20L)** 100,000 cells; **(20M-20R)** 175,000; and **(20S-20X)** 250,000. Days cultured: 1 day **(20A, 20G, 20M and 20S);** 3 days **(20B, 20H, 20N and 20T);** 4 days **(20C, 20I, 20O and 20U);** 5 days **(20D, 20J, 20P and 20V);** 7 days **(20E, 20K, 20Q and 20W);** and 10 days **(20F, 20L, 20R and 20X).**

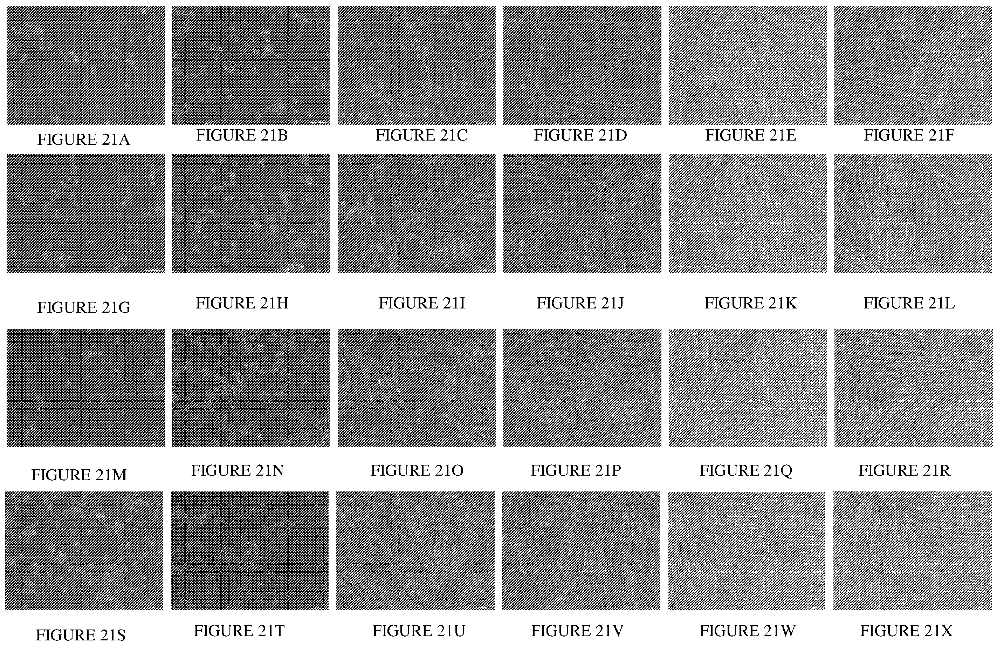

**Figs. 21A-21X** is micrographs of 2D myotube formation of RN-A56. Scale bar: 100 µm. Cell number: **(21A-21F)** 50,000 cells; **(21G-21L)** 100,000 cells; **(21M-21R)** 175,000; and **(21S-21X)** 250,000. Days cultured: 1 day **(21A, 21G, 21M and 21S);** 3 days **(21B, 21H, 21N and 21T);** 4 days **(21C, 21I, 21O and 21U);** 5 days **(21D, 21J, 21P and 21V);** 7 days **(21E, 21K, 21Q and 21W);** and 10 days **(21F, 21L, 21R and 21X).**

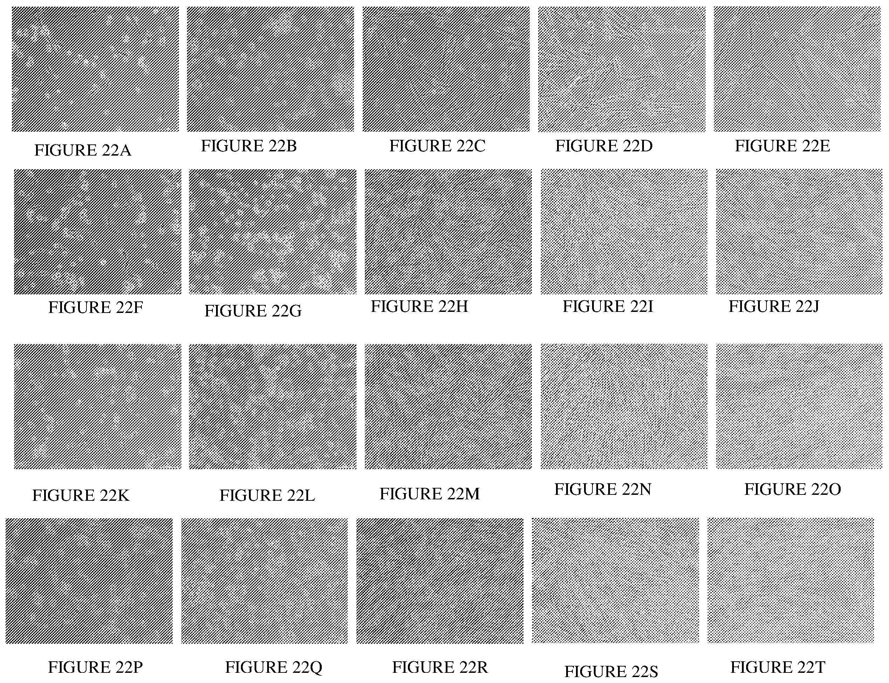

**Figs. 22A-22T** is micrographs of 2D myotube formation of PB-A34. Scale bar:100 µm. Cell number: **(22A-22E)** 50,000 cells; **(22F-22J)** 100,000 cells; **(22K-22O)** 175,000; and **(22P-22T)** 250,000. Days cultured: 1 day **(22A, 22F, 22K and 22P);** 2 days **(22B, 22G, 22L and 22Q);** 4 days **(22C, 22H, 22M and 22R);** 7 days **(22D, 22I, 22N and 22S);** and 10 days **(22E, 22J, 22O and 22T).**

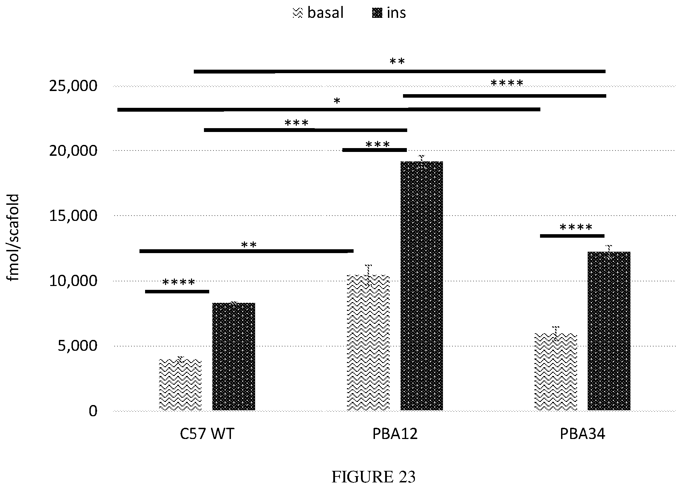

**Fig. 23** is a vertical bar graph showing 2-DOG uptake rates in C57-SC myotubes grown 1 week in 2D culture. * p<0.05, ** p<0.01, *** p<0.001, **** p<0.0001.

### DETAILED DESCRIPTION OF THE INVENTION

The present invention relates to a transplantable scaffold-cell construct comprising: (a) a porous scaffold comprising at least one biocompatible polymer comprising a combination of poly-l-lactic acid (PLLA) and polylactic glycolic acid (PLGA), or collagen; and (b) a cell population deposited on or in scaffold comprising recombinant myoblasts: (i) comprising an exogenous polynucleotide encoding glucose transporter type 4 (GLUT4); (ii) over-expressing the GLUT4; and (iii) having increased GLUT4 activity, compared to control wild type myoblast cells, such as for restoring glucose homoeostasis.

The present invention is based, in part, on the finding that biocompatible scaffolds seeded with cells over-expressing GLUT4, restored normoglycemia. As demonstrated hereinbelow, scaffold-cell constructs specifically overexpressing GLUT4 were found to have greater glucose uptake rates compared with control cells *in-vitro.* As exemplified herein, glucose uptake rate was further enhanced when scaffold-cell constructs specifically over-expressing GLUT4 were incubated in the presence of insulin *in-vitro.* The invention is further based, in part, on the finding that scaffold-cell constructs specifically expressing GLUT4 restored normoglycemia when transplanted into insulin-resistant mice, thereby eliciting a therapeutic avenue relevant for treating diabetes and metabolic syndrome.

As used herein, the terms "treat," "treating," "treatment," and the like refer to reducing or ameliorating a disease or condition, e.g., diabetes, hyperglycemia, insulin resistance, and/or symptoms associated therewith. In some embodiments, treatment includes the partial or complete regeneration of normoglycemia in a subject. It will be appreciated that, although not precluded, treating a disease or condition does not require that the disease, condition, or symptoms associated therewith be completely eliminated.

As used herein, the term "normoglycemia" refers to the normal levels of glucose in the blood of healthy people. Normoglycemia is glucose levels of about 70 to 100 milligrams per deciliter (mg/dL) of blood in healthy people pre-meal (e.g., fasting). Normoglycemia is glucose levels of about 75 to 100 mg/dL of blood in healthy people pre-meal (e.g., fasting). Normoglycemia is glucose levels of about 85 to 100 mg/dL of blood in healthy people pre-meal (e.g., fasting). Normoglycemia is glucose levels of about 70 to 95 mg/dL of blood in healthy people pre-meal (e.g., fasting). Normoglycemia is glucose levels of about 75 to 90 mg/dL of blood in healthy people pre-meal (e.g., fasting).

Normoglycemia is glucose levels up to 140 mg/dL of blood in healthy people postprandial (e.g., 2 hours after eating). Normoglycemia is glucose levels of about 80 to 140 mg/dL of blood in healthy people postprandial (e.g., after eating). Normoglycemia is glucose levels of about 90 to 140 mg/dL of blood in healthy people postprandial (e.g., after eating). Normoglycemia is glucose levels of about 100 to 140 mg/dL of blood in healthy people postprandial (e.g., after eating). Normoglycemia is glucose levels of about 110 to 140 mg/dL of blood in healthy people postprandial (e.g., after eating). Normoglycemia is glucose levels of about 120 to 140 mg/dL of blood in healthy people postprandial (e.g., after eating). Normoglycemia is glucose levels of about 130 to 140 mg/dL of blood in healthy people postprandial (e.g., after eating). Normoglycemia is glucose levels less or equal to 140 mg/dL of blood in healthy people postprandial (e.g., 2 hours after eating).

The terms "glucose homeostatic levels", "normoglycemia" and "normoglycemia" are interchangeable.

As used herein, the term "impaired fasting glucose (IFG)" refers to fasting glucose levels between 100-125 mg/dL.

In another embodiment, a glucose level lower than the mentioned herein normoglycemia is hypoglycemia. In another embodiment, a glucose level greater than the mentioned herein normoglycemia is considered hyperglycemia. In another embodiment, a subject having fasting blood glucose level of 100-125 mg/dL or 2 hours postprandial (e.g. test by glucose tolerance test) level of 140-199 mg/dL, is considered pre-diabetic and/or having insulin resistance.

Hyperglycemia of 200 mg/dL and above, without returning to basal levels within a period of 2 hours after a glucose tolerance test of 75 gr, is indicative of diabetes.

Glucose levels are measured by means of a blood test after fasting (e.g., FGT). In some embodiments, glucose levels are measured by means of an oral glucose tolerance test (e.g., OGTT). Glucose levels are estimated by the level of glycosylated hemoglobin (e.g., HbA_{1C}). As apparent to one skilled in the art, normoglycemia is having up to 5.7% HbA_{1C}. Hyperglycemia is having HbA_{1C} level of 6.5% or more. HbA_{1C} level of 5.7-6.4% is indicative of prediabetes. In another embodiment, HbA_{1C} level of 6.5% or more is indicative of diabetes.

### Cells over-expressing glucose transporters

In some embodiments, the invention provides a scaffold-cell constructs comprising: (a) a porous scaffold comprising at least one biocompatible polymer comprising a combination of poly-l-lactic acid (PLLA) and polylactic glycolic acid (PLGA), or collagen; and; and (b) a cell population deposited on or in the scaffold comprising recombinant myoblasts: (i) comprising an exogenous polynucleotide encoding glucose transporter type 4 (GLUT4); (ii) over-expressing said GLUT4; and (iii) having increased GLUT4 activity, compared to control wild type myoblast cells, and wherein the scaffold-cell construct is transplantable.

In some embodiments, the human GLUT 4 comprises a polynucleotide sequence according to accession number M20747.1. In some embodiments, the human GLUT 4 comprises a polypeptide sequence according to accession number AAA59189.1. In some embodiments, the murine GLUT 4 comprises a polynucleotide sequence according to accession number AB008453.1. In some embodiments, the human GLUT 4 comprises a polypeptide sequence according to accession number BAB03251.1.

In some embodiments, the cell population deposited on or in the scaffold comprises solitary recombinant myoblast cells. In some embodiments, the cell population deposited on or in the scaffold comprises at least 60%, 70%, 80%, 85%, 90% or 99% solitary recombinant myoblast cells, and any value and range therebetween. In some embodiments, the cell population deposited on or in the scaffold comprises 40-60%, 50-70%, 60-80%, 65-85%, 70-90% or 80-99% solitary recombinant myoblast cells. Each possibility represents a separate embodiment of the invention.

In some embodiments, the cell population of the present invention comprises recombinant myoblasts expressing GLUT4 levels sufficient for maintaining glucose homeostasis at levels of less than or equal to 100 mg/dL at fasting. In another embodiment, the cell population comprises recombinant myoblasts expressing GLUT4 levels sufficient for maintaining glucose homeostasis at levels of less than or equal to 110, 120 or 130 mg/dL at fasting. In another embodiment, fasting is for at least 1, 4, 8, 12 or 14 hours, and any range and value therebetween. In another embodiment, fasting is for 1-3 h, 2-5 h, 3-8 h, 4-6 h, 4-9 h, 7-12 h, 8-16 h, 14-20 h, 12-24 h. Each possibility represents a separate embodiment of the invention.

In some embodiments, the cell population of the present invention deposited on or in the scaffold comprises recombinant myoblasts expressing GLUT4 levels sufficient for maintaining glucose homeostasis at levels of less than 140 mg/dL postprandial. In another embodiment, the cell population deposited on or in the scaffold comprises recombinant myoblasts expressing GLUT4 levels sufficient for maintaining glucose homeostasis at levels of less than 150 or 160 mg/dL postprandial. In another embodiment, postprandial is not more than 15, 30, 45 or 60 min postprandial, and any value and range therebetween. In another embodiment, postprandial is not more than 2, 3, 4, 5 or 6 hours postprandial, and any value and range therebetween. In another embodiment, postprandial is 0.5-1.5 h, 1-3 h, 2-4 h, 3-5 h, or 3-7 h postprandial. Each possibility represents a separate embodiment of the invention.

In some embodiments, the cell population of the present invention deposited on or in the scaffold comprises recombinant myoblasts having increased glucose responding activity or glucose sensitivity compared to control cells. In some embodiments, the cell population deposited on or in the scaffold comprises recombinant myoblasts having increased glucose responding activity or glucose sensitivity compared to control wild type myoblast cells in the absence of insulin (e.g., basal). In some embodiments, the cell population deposited on or in the scaffold comprises recombinant myoblasts having increased glucose responding activity or glucose sensitivity compared to control wild type myoblast cells in the presence of insulin. In some embodiments, the cell population deposited on or in the scaffold comprises recombinant myoblasts having increased glucose responding activity or glucose sensitivity compared to control wild type myoblast cells in the absence or presence of insulin. In some embodiments, the increased glucose responding activity or glucose sensitivity of the cell population deposited on or in the scaffold comprising recombinant myoblasts results in increased rates of glucose uptake, increased amounts of glucose taken by the recombinant myoblast cells, increased rates of glucose metabolism, increased amounts of metabolized glucose, and any combination thereof, all compared to control wild type myoblast cells under basal conditions (i.e., in the absence of insulin) or in the presence of insulin, or any combination thereof.

As would be apparent to one skilled in the art, fully differentiated skeletal muscle cells are characterized by positive immune-stain for myoblasts' differentiation and structural markers. Non-limiting examples of myoblasts' differentiation markers include MYOG (differentiation marker), and desmin and MyH (structural markers). As well known to a skilled artisan, fully differentiated skeletal muscle cells are observed by microscopy as organized multinucleated myotubes.

In another embodiment, a cell population of the invention deposited on the scaffold comprises recombinant myoblasts exhibiting insulin-responsive properties. In another embodiment, the insulin responsive properties are enhanced glucose uptake. In another embodiment, glucose uptake is enhanced by at least by 2-fold, 3-fold, 4-fold, 5-fold, 6-fold, 8-fold or 10-fold, and any value and range therebetween. In some embodiments, glucose uptake is enhanced by 50-100%, 100-250%, 200-500%, 300-700%, 650-900%, or 750-1,500%. Each possibility represents a separate embodiment of the invention.

In some embodiments, cellular glucose uptake is attributed to the activity of GLUT4.

As used herein, "increased GLUT4 activity" includes, but not is limited to, increase of any one of: GLUT4 gene expression, GLUT4 cellular content, membrane translocation by the cellular translocation machinery pathway, insulin signal transduction, glucose sensitivity in the absence or presence of insulin, or any combination thereof. In some embodiments, increased gene expression includes, but is not limited to, increased amount of the gene's mRNA molecules, increased amount of the translated polypeptides, or any combination thereof. In some embodiments, increased GLUT4 activity enhances cellular insulin sensitivity. In some embodiments, increased GLUT4 activity enhances cellular glucose uptake. In some embodiments, increased GLUT4 activity enhances cellular insulin sensitivity and cellular glucose uptake.

Insulin signal transduction inhibits GLUT4 degradation. GLUT4 is degraded by a proteasome dependent pathway. GLUT4 is degraded by an oxidative stress mediated pathway. Degradation of GLUT4 can be determined by any method known in the art, including, but not limited to, methods utilizing specific anti GLUT4 antibodies, comprising anti ubiquitinated-GLUT4 antibodies, among others. Non-limiting examples of methods which utilize antibodies include, but are not limited to, sandwich enzyme linked immunosorbent assay (ELISA, e.g., of either tissue homogenates, cell lysate or other biological fluids), 26S proteasome degradation assay, immunoprecipitation, immuneblotting, immune-histochemistry, immune-cytochemistry, any combination thereof, or any other method known to one of ordinary skill in the art.

In some embodiments, GLUT4 activity is increased in the cell population deposited in or on the scaffold comprising recombinant myoblasts by at least 2-fold, 5-fold, 10-fold, 25-fold or 100-fold, and any value and range therebetween. In another embodiment, GLUT4 activity is increased in the cell population deposited in or on the scaffold comprises recombinant myoblasts by 5-50%, 20-100%, 75-250%, 200-500%, 450-750%, or 600-1,000%. Each possibility represents a separate embodiment of the invention.

As used herein, the term "recombinant cell", refers to a cell whose genetic composition was modified. In one embodiment, a recombinant cell comprises exogenous polynucleotide. In another embodiment, a recombinant cell expresses an exogenous polynucleotide. In one embodiment, a recombinant cell constitutively expresses an endogenous polynucleotide. In another embodiment, a recombinant cell conditionally expresses an endogenous polynucleotide. A non-limiting example of constitutive expression is achieved by contacting a cell with an endogenous polynucleotide operably linked to a constantly operating promoter polynucleotide. In another embodiment, recombinant cell facultatively expresses exogenous polynucleotide in response to a specific stimulation (e.g., induced or conditional expression). In another embodiment, recombinant cell expresses exogenous polynucleotide indefinitely. Recombinant expressions systems are well known to one skilled in the art, non-limiting examples of which include the Tetracycline-controlled transcriptional activation ("Tet-on/Tet off"), Actin-GAL4-UAS, IPTG-inducible conditional expression, or others.

In another embodiment, the expression of the GLUT4 gene in the cell population deposited in or on the scaffold comprising recombinant myoblasts of the invention is upregulated by at least 2-fold, 3-fold, 4-fold, 5-fold, 6-fold, 8-fold or 10-fold, and any value and range therebetween. In another embodiment, the expression of the GLUT4 gene in the cell population deposited in or on the scaffold comprising recombinant myoblasts of the invention is upregulated by 5-50%, 40-100%, 75-250%, 200-350%, 300-500%, 400-750%, or 700-1,500%. Each possibility represents a separate embodiment of the invention.

In another embodiment, increased GLUT4 activity is in the level sufficient to restore glucose homeostasis. In another embodiment, increased GLUT4 activity is in the level sufficient to maintain glucose homeostasis. In another embodiment, increased GLUT4 activity is in the level sufficient to rectify glucose homeostasis. In another embodiment, increased GLUT4 activity is in the level sufficient to reduce hyperglycemia in a subject. In another embodiment, increased GLUT4 activity is in the level sufficient to restore normoglycemia in a subject afflicted with hyperglycemia. In another embodiment, increased GLUT4 activity is in the level sufficient to reduce fasting glucose levels to levels less than or equal to 80 mg/dL in a subject. In another embodiment, increased GLUT4 activity is in the level sufficient to reduce fasting glucose levels to levels less than or equal to 90 mg/dL in a subject. In another embodiment, increased GLUT4 activity is in the level sufficient to reduce fasting glucose levels to levels less than or equal to 95 mg/dL in a subject. In another embodiment, increased GLUT4 activity is in the level sufficient to reduce fasting glucose levels to levels less than or equal to 100 mg/dL in a subject. In another embodiment, increased GLUT4 activity is in the level sufficient to reduce fasting glucose levels to levels less than or equal to 105 mg/dL in a subject. In another embodiment, increased GLUT4 activity is in the level sufficient to reduce fasting glucose levels to levels less than or equal to 110 mg/dL in a subject. In another embodiment, increased GLUT4 activity is in the level sufficient to reduce fasting glucose levels to levels less than or equal to 115 mg/dL in a subject. In another embodiment, increased GLUT4 activity is in the level sufficient to reduce fasting glucose levels to levels less than or equal to 120 mg/dL in a subject. In another embodiment, increased GLUT4 activity is in the level sufficient to reduce postprandial glucose levels to levels less than 135 mg/dL in a subject. In another embodiment, increased GLUT4 activity is in the level sufficient to reduce postprandial glucose levels to levels less than 140 mg/dL in a subject. In another embodiment, increased GLUT4 activity is in the level sufficient to reduce postprandial glucose levels to levels less than 145 mg/dL in a subject. In another embodiment, increased GLUT4 activity is in the level sufficient to reduce postprandial glucose levels to levels less than 150 mg/dL in a subject. In another embodiment, increased GLUT4 activity is in the level sufficient to reduce postprandial glucose levels to levels less than 155 mg/dL in a subject. In another embodiment, increased GLUT4 activity is in the level sufficient to reduce postprandial glucose levels to levels less than 160 mg/dL in a subject.

In some embodiments, a recombinant myoblast cell of the present invention comprises a myoblast cell in which GLUT4 levels have been directly elevated. As used herein, the term "directly elevated levels of GLUT4" refers to contacting a myoblast cell with an exogenous polynucleotide comprising a GLUT4 encoding sequence and inducing its expression, thereby resulting in its elevated levels in the recombinant cell. In some embodiments, the elevated levels are increased levels of the GLUT4 encoding gene transcription. In some embodiments, the elevated levels are increased amounts of the GLUT4 mRNA molecules. In some embodiments, the elevated levels are increased rates of the GLUT4 mRNA translation. In some embodiments, the elevated levels are increased amounts of the GLUT4 polypeptide. In some embodiments, the elevated levels are achieved by a vector or a plasmid transfection. In some embodiments, the vector or plasmid is transfected to a myoblast cell according to the invention. In some embodiments, the vector comprises the exogenous polynucleotide comprising GLUT4 encoding sequence.

The term "polynucleotide" refers to a nucleic acid (e.g., DNA or RNA) sequence that comprises coding sequences necessary for the production of a polypeptide. In one embodiment, a polynucleotide refers to a single or double stranded nucleic acid sequence which is isolated and provided in the form of an RNA sequence, a complementary polynucleotide sequence (cDNA), a genomic polynucleotide sequence and/or a composite polynucleotide sequences (e.g., a combination of the above).

In one embodiment, "complementary polynucleotide sequence" refers to a sequence, which results from reverse transcription of messenger RNA using a reverse transcriptase or any other RNA dependent DNA polymerase. In one embodiment, the sequence can be subsequently amplified in vivo or in vitro using a DNA polymerase.

In one embodiment, "genomic polynucleotide sequence" refers to a sequence derived (isolated) from a chromosome and thus it represents a contiguous portion of a chromosome.

As an example, a vector or a plasmid is a composite vector or plasmid. A vector or a plasmid is a man-made vector or plasmid comprising at least one DNA sequence which is artificial. Examples for vector or a plasmid include: pcDNA3, pcDNA3.1(+/-), pGL3, pZeoSV2(+/-), pSecTag2, pDisplay, pEF/myc/cyto, pCMV/myc/cyto, pCR3.1, pSinRep5, DH26S, DHBB, pNMT1, pNMT41, pNMT81, which are available from Invitrogen, pCI which is available from Promega, pMbac, pPbac, pBK-RSV and pBK-CMV which are available from Strategene, pTRES which is available from Clontech, and their derivatives.

A vector or a plasmid comprises regulatory elements from eukaryotic viruses such as retroviruses are used by the present invention. SV40 vectors include pSVT7 and pMT2. For example, vectors derived from bovine papilloma virus include pBV-1MTHA, and vectors derived from Epstein Bar virus include pHEBO, and p2O5. Other exemplary vectors include pMSG, pAV009/A+, pMTO10/A+, pMAMneo-5, baculovirus pDSVE, and any other vector allowing expression of proteins under the direction of the SV-40 early promoter, SV-40 later promoter, metallothionein promoter, murine mammary tumor virus promoter, Rous sarcoma virus promoter, polyhedrin promoter, or other promoters shown effective for expression in eukaryotic cells.

Various methods can be used to introduce the expression vector of the present invention into cells. Such methods are generally described in Sambrook et al., Molecular Cloning: A Laboratory Manual, Cold Springs Harbor Laboratory, New York (1989, 1992), in Ausubel et al., Current Protocols in Molecular Biology, John Wiley and Sons, Baltimore, Md. (1989), Chang et al., Somatic Gene Therapy, CRC Press, Ann Arbor, Mich. (1995), Vega et al., Gene Targeting, CRC Press, Ann Arbor Mich. (1995), Vectors: A Survey of Molecular Cloning Vectors and Their Uses, Butterworths, Boston Mass. (1988) and Gilboa et at. [Biotechniques 4 (6): 504-512, 1986] and include, for example, stable or transient transfection, lipofection, electroporation and infection with recombinant viral vectors. In addition, see U.S. Patent Nos. 5,464,764 and 5,487,992 for positive-negative selection methods.

Typically, introduction of nucleic acid by viral infection offers several advantages over other methods such as lipofection and electroporation, since higher transfection efficiency can be obtained due to the infectious nature of viruses.

In another embodiment, the cell population deposited in or on the scaffold comprising recombinant myoblasts occupies the scaffold in all three dimensions. In another embodiment, the cell population deposited in or on the scaffold comprising recombinant myoblasts occupies the pores. In another embodiment, the cell population deposited in or on the scaffold comprising recombinant myoblasts resides within a pore. In another embodiment, the cell population deposited in or on the scaffold comprising recombinant myoblasts resides on the scaffold's surface. In another embodiment, the cell population deposited in or on the scaffold comprising recombinant myoblasts is present both within the pores and on the scaffold's surface. In some embodiments, recombinant myoblast cells of the cell population as mentioned herein occupies at least 25%, at least 35%, at least 45%, at least 60%, at least 75%, at least 85%, at least 90%, at least 95%, or at least 99% of areas of the scaffold as disclosed herein, for example, pores, surface, or both, and any value and range therebetween. In some embodiments, recombinant myoblast cells of the cell population as mentioned herein occupies 20-30%, 25-35%, 25-45%, 40-60%, 50-75%, 70-85%, 75-90%, 80-95%, 92-100% of areas of the scaffold as disclosed herein, for example, pores, surface, or both. Each possibility represents a separate embodiment of the invention.

In another embodiment, the cells of the cell population comprising the recombinant myoblast cells are autologous cells. In another embodiment, the cells of the cell population comprising the recombinant myoblast cell are allogeneic cells.

In another embodiment, the cell population deposited in or on the scaffold comprises recombinant myoblasts expressing one or more markers selected from desmin, myosin heavy chain (MYH), or myogenin (MYOG). None limiting examples for methods for detecting such markers are disclosed hereinbelow and would be apparent to a skilled artisan.

### Scaffolds

As used herein, the term "scaffold" refers to a structure comprising a biocompatible material that provides a surface suitable for adherence, attachment, anchoring, maturation, differentiation, proliferation, or any combination thereof, of cells. A scaffold may further provide mechanical stability and support. A scaffold may be in a particular shape or form so as to influence or delimit a three-dimensional shape or form assumed by a population of proliferating cells. As used herein three-dimensional shapes include: films, ribbons, cords, sheets, flat discs, cylinders, spheres, 3-dimensional amorphous shapes, or others.

As used herein, the term "biocompatible", in some embodiments, refers to the ability of an object to be accepted by and to function in a recipient without eliciting a significant foreign body response (such as, for a non-limiting example, an immune, inflammatory, thrombogenic, or the like response). For example, when used with reference to one or more of the polymeric materials of the invention, biocompatible refers to the ability of the polymeric material (or polymeric materials) to be accepted by and to function in its intended manner in a recipient.

The scaffold, in one embodiment, is a porous matrix. In another embodiment, the porous scaffold comprises at least 50% porosity. In another embodiment, the porous scaffold comprises at least 60% porosity, at least 70% porosity, at least 75% porosity, at least 80% porosity, at least 85% porosity, at least 90% porosity, at least 92% porosity, or at least 95% porosity, and any value and range therebetween. In another embodiment, the porous scaffold comprises 45-55% porosity, 50-70% porosity, 60-80% porosity, 75-90% porosity, or 80-97% porosity. Each possibility represents a separate embodiment of the invention.

In another embodiment, the porous scaffold comprises pores having a diameter of at least 100 µm. In another embodiment, the porous scaffold comprises pores having a diameter of at least 120 µm. In another embodiment, the porous scaffold comprises pores having a diameter of at least 150 µm. In another embodiment, the porous scaffold comprises pores having a diameter of 100-900 µm. In another embodiment, the porous scaffold comprises pores having a diameter of 120-900 µm. In another embodiment, the porous scaffold comprises pores having a diameter of 120-850 µm. In another embodiment, the porous scaffold comprises pores having a diameter of 150-800 µm. In another embodiment, the porous scaffold comprises pores having a diameter of 200-800 µm. In another embodiment, the porous scaffold comprises pores having a diameter of 220-750 µm.

In another embodiment, the scaffold (e.g., matrix) is devoid of any one of an organized structure, layer, or network of layers. In another embodiment, the composition is devoid of any layer of aligned fibers. In another embodiment, the scaffold is devoid of any layer of aligned fibers. In another embodiment, the composition is devoid of curved fibers. In another embodiment, the scaffold is devoid of curved fibers.

In another embodiment, the scaffold described herein comprises both poly-l-lactic acid (PLLA) and polylacticglycolicacid (PLGA), or collagen. In another embodiment, PLLA and PLGA are in 1:3 to 3:1 w/w ratio. In another embodiment, PLLA and PLGA are in 1:2 to 2:1 w/w ratio. In another embodiment, PLLA and PLGA are in 1:1.5 to 1.5:1 w/w ratio. In another embodiment, PLLA and PLGA are in 1:1 w/w ratio.

In another embodiment, the present invention is further directed to a composition that is cultured for at least 7 days. In another embodiment, the composition is cultured for at least 14 days. In another embodiment, the composition is cultured for at least 21 days. In another embodiment, the composition is cultured for at least 28 days. In another embodiment, the composition is cultured for at least 3 months days. In another embodiment, the composition is cultured for 7-15 days, 14-28 days, 21-35 days, 1-2 months, 2-3 months, or 4-6 months. Each possibility represents a separate embodiment of the invention.
In some embodiments, the scaffolds described herein includes a therapeutic agent comprising glucose transporter type 4 (GLUT4), also known as either facilitated glucose transporter member 4 or solute carrier family 2.

The porosity of the scaffold is controlled by a variety of techniques known to those skilled in the art.

The choice of polymer and the ratio of polymers in a co-polymer scaffold of the invention is adjusted to optimize the stiffness/porosity of the scaffold. The molecular weight and cross-link density of the scaffold is regulated to control both the mechanical properties of the scaffold and the degradation rate (for degradable scaffolds). The mechanical properties are optimized to mimic those of the tissue at the implant site. The shape and size of the final scaffold are adapted for the implant site and tissue type. Scaffold materials comprise natural or synthetic organic polymers that can be gelled, or polymerized or solidified (e.g., by aggregation, coagulation, hydrophobic interactions, or cross-linking) into a hydrogel e.g., structure that entraps water and/or other molecules.

In another embodiment, polymers used in scaffold material compositions are biocompatible and act as adhesive substrates for cells.

In another embodiment, the structural scaffold materials are non-resorbing or non-biodegradable polymers or materials. The term "non-biodegradable polymer", as used herein, refers to a polymer or polymers which at least substantially (i.e., 50% or more) do not degrade or erode in-vivo. The terms "non-biodegradable" and "non-resorbing" are equivalent and are used interchangeably herein.

In another embodiment, scaffold material comprises collagen.

In another embodiment, the scaffolds of the invention are made by any of a variety of techniques known to those skilled in the art. Salt-leaching, porogens, solid-liquid phase separation (sometimes termed freeze-drying), and phase inversion fabrication are used, in some embodiments, to produce porous scaffolds.

As used herein, "transplanting" refers to providing the scaffold supported recombinant myoblast cells of the present invention (e.g., scaffold-cell construct), using any suitable route, as known to one skilled in the art. In one embodiment, the scaffold supported recombinant myoblast cells are administered by injection using a catheter.

In another embodiment, recombinant myoblast cells on scaffolds induce insulin sensitivity behavior, including maintaining proper glucose homeostasis. As exemplified herein, the composition comprising PLLA/PLGA or collagen scaffolds seeded with GLUT4 overexpressing recombinant myoblast cells dramatically and unexpectedly increased the therapeutic potential of transplantation in patients afflicted with a metabolic syndrome, including but not limited to obesity, pre-diabetes, Diabetes mellitus type 2, insulin resistance or related to insulin resistance, among others.

In another embodiment, the invention further provides use of scaffolds comprising a cell population deposited on or in the scaffold comprising recombinant myoblasts cells as described herein for ameliorating insulin-desensitization, glucose dysregulation, hyperglycemia or any combination thereof, using.

In another embodiment, the invention provides a glucose homeostasis rectifying cell population containing GLUT4 overexpressing recombinant myoblast cells seeded on a scaffold as described herein. In some embodiments, use of the scaffold cell construct in transplantation into a subject in need thereof, including but not limited to a subject afflicted with a diabetes, provides a glucose homeostasis rectifying effect.

The phrase "treating" refers to inhibiting or arresting the development of a disease, disorder or condition and/or causing the reduction, remission, or regression of a disease, disorder or condition in an individual suffering from, or diagnosed with, the disease, disorder or condition. Those of skill in the art will be aware of various methodologies and assays which can be used to assess the development of a disease, disorder or condition, and similarly, various methodologies and assays which can be used to assess the reduction, remission or regression of a disease, disorder or condition.

The term "subject" or "patient" refers to an animal which is the object of treatment, observation, or experiment. By way of example only, a subject includes, but is not limited to, a mammal, including, but not limited to, a human or a non- human mammal. Non-limiting examples of a non-human mammal include, primate, murine, bovine, equine, canine, ovine, or feline subject.

The term "biological sample" refers to a sample obtained from a subject, including, but not limited to, sample of biological tissue or fluid origin obtained in vivo or in vitro. Such samples can be, but are not limited to, body or bodily fluid (e.g., blood, blood plasma, serum, or urine), organs, tissues, fractions thereof, and cells isolated from mammals including, humans. Biological samples also may include sections of the biological sample including tissues (e.g., sectional portions of an organ or tissue). Biological samples may also include extracts from a biological sample, for example, an antigen from a biological fluid (e.g., blood or urine). In some embodiments, the biological sample is blood, tear, saliva, sweat, or urine. In some embodiments, the biological sample is obtained by noninvasive techniques, for example, from tears, saliva, sweat, or urine.

According to some embodiments, a method or a process of making a scaffold-cell construct for restoring glucose homeostatic levels in a diabetic subject in need thereof, is provided. In some embodiments, the method or process are directed to a scaffold-cell construct restoring glucose homeostatic levels in a diabetic subject in need thereof, of: less than or equal to 100 mg/dL at fasting; and less than 140 mg/dL postprandial. In some embodiments, the method or process is directed to a scaffold-cell construct restoring glucose homeostatic levels in a diabetic subject in need thereof, of: less than or equal to 120 mg/dL at fasting; and less than 160 mg/dL postprandial.

In some embodiments, the method or process comprises contacting a recombinant myoblast cell : (i) comprising an exogenous polynucleotide encoding GLUT4; (ii) over-expressing said GLUT4; and (iii) having increased GLUT4 activity; and (iii) having increased GLUT4 activity, compared to control wild type myoblast cells, with a scaffold comprising at least one biocompatible polymer comprising a combination of PLLA and PLGA or collagen, , as mentioned hereinabove and such as exemplified herein.

As used herein, at least a portion comprises: at least 10%, at least 20%, at least 30%, at least 40%, at least 50%, at least 60%, at least 70%, at least 80%, at least 90%, or at least 95% of the first cell population, and any value and range therebetween. In some embodiments, a portion comprises 10-30%, 20-40%, 30-50%, 40-60%, 50-70%, 60-80%, 70-90%, or 80-100% if the first cell population. Each possibility represents a separate embodiment of the invention.

### Treatment and pharmaceutical compositions

According to some embodiments, a pharmaceutical composition comprising scaffold-cell constructs and a pharmaceutically acceptable carrier, excipient, or adjuvants is provided. In some embodiments, the pharmaceutical composition comprises a therapeutically effective amount of the scaffold-cell construct comprising: (a) a porous scaffold comprising at least one biocompatible polymer comprising a combination of poly-l-lactic acid (PLLA) and polylactic glycolic acid (PLGA), or collagen; and (b) a cell population deposited on or in said scaffold comprising recombinant myoblasts: (i) comprising an exogenous polynucleotide encoding glucose transporter type 4 (GLUT4); (ii) over-expressing said GLUT4; and (iii) having increased GLUT4 activity, compared to control wild type myoblast cells, for use in reducing glucose levels in a subject. In some embodiments, the composition is for use in reducing glucose levels to less than or equal to 100 mg/dL at fasting; and less than 140 mg/dL postprandial. In some embodiments, the pharmaceutical composition comprises a therapeutically effective amount of the scaffold-cell constructs over-expressing GLUT4, for use in reducing glucose levels in a subject. In some embodiments, the composition is for use in reducing glucose levels to less than or equal to 120 mg/dL at fasting; and less than 160 mg/dL postprandial.

In some embodiments, the pharmaceutical composition is for use in treating diabetes or other metabolic syndrome(s).

As used herein, the term "metabolic syndrome, disease, disorder, or condition" refers to any disease or disorder characterized by excess abdominal fat, hypertension, abnormal fasting plasma glucose level or insulin resistance, high triglyceride levels, low high-density lipoprotein (HDL) cholesterol level, and any combination thereof. In some embodiments, the metabolic syndrome disorders which can be treated according to the present invention are diverse and will be easily understood by the skilled artisan. Without any limitation mentioned are obesity, pre-diabetes, diabetes, hyperglycemia, diabetic dyslipidemia, hyperlipidemia, hypertriglyceridemia, hyper-fattyacidemia, hypercholesterolemia, hyperinsulinemia, insulin-resistance or insulin-resistance related. High risks of metabolic syndrome disease include, but are not limited to, obstructive sleep apnea, nonalcoholic steatohepatitis, chronic kidney disease, polycystic ovary syndrome and low plasma testosterone, erectile dysfunction, or both.

According to some embodiments, the use of the scaffold-cell construct of the present invention for preparation of a medicament for treating metabolic syndrome is provided.

As used herein, the terms "carrier", "adjuvant" or "excipient" refer to any component of a pharmaceutical composition that is not the active agent. As used herein, the term "pharmaceutically acceptable carrier" refers to non-toxic, inert solid, semi-solid liquid filler, diluent, encapsulating material, formulation auxiliary of any type, or simply a sterile aqueous medium, such as saline. Suitable pharmaceutically acceptable carriers, excipients, and diluents in this regard are well known to those of skill in the art, such as those described in The Merck Index, Thirteenth Edition, Budavari et al., Eds., Merck & Co., Inc., Rahway, N.J. (2001); the CTFA (Cosmetic, Toiletry, and Fragrance Association) International Cosmetic Ingredient Dictionary and Handbook, Tenth Edition (2004); and the "Inactive Ingredient Guide," U.S. Food and Drug Administration (FDA) Center for Drug Evaluation and Research (CDER) Office of Management. Examples of pharmaceutically acceptable excipients, carriers and diluents that may be useful in the present compositions include distilled water, physiological saline, Ringer's solution, dextrose solution, Hank's solution, and DMSO. These additional inactive components, as well as effective formulations and administration procedures, are well known in the art and are described in standard textbooks, such as Goodman and Gillman's: The Pharmacological Bases of Therapeutics, 8th Ed., Gilman et al. Eds. Pergamon Press (1990); Remington's Pharmaceutical Sciences, 18th Ed., Mack Publishing Co., Easton, Pa. (1990); and Remington: The Science and Practice of Pharmacy, 21st Ed., Lippincott Williams & Wilkins, Philadelphia, Pa., (2005).

The carrier may comprise, in total, from about 0.1% to about 99.99999% by weight of the pharmaceutical compositions presented herein.

As used herein, the terms "therapeutically active molecule" or "therapeutic agent" mean a molecule, group of molecules, complex or substance administered to an organism for diagnostic, therapeutic, preventative medical, or veterinary purposes. This term includes pharmaceuticals, e.g., small molecules, treatments, remedies, biologics, devices, and diagnostics, including preparations useful in clinical screening, prevention, prophylaxis, healing, imaging, therapy, surgery, monitoring, and the like. This term can also specifically include nucleic acids and compounds comprising nucleic acids that produce a bioactive effect, for example.

The term "therapeutically effective amount" refers to the concentration of cells within a scaffold-cell construct that over-express GLUT4 and are normalized to body weight (BW) that is effective to treat a disease or disorder in a mammal. The term "a therapeutically effective amount" refers to an amount effective, at dosages and for periods of time necessary, to achieve the desired therapeutic or prophylactic result. A physician or veterinarian of ordinary skill can readily determine and prescribe the effective amount of the bioactive agent required.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

It should be understood that the terms "a" and "an" as used above and elsewhere herein refer to "one or more" of the enumerated components. It will be clear to one of ordinary skill in the art that the use of the singular includes the plural unless specifically stated otherwise. Therefore, the terms "a", "an" and "at least one" are used interchangeably in this application.

For purposes of better understanding the present teachings and in no way limiting the scope of the teachings, unless otherwise indicated, all numbers expressing quantities, percentages or proportions, and other numerical values used in the specification and claims, are to be understood as being modified in all instances by the term "about". Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques.

In the description and claims of the present application, each of the verbs, "comprise," "include" and "have" and conjugates thereof, are used to indicate that the object or objects of the verb are not necessarily a complete listing of components, elements or parts of the subject or subjects of the verb.

Other terms as used herein are meant to be defined by their well-known meanings in the art.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination or as suitable in any other described embodiment of the invention. Certain features described in the context of various embodiments are not to be considered essential features of those embodiments, unless the embodiment is inoperative without those elements.

### EXAMPLES

Generally, the nomenclature used herein, and the laboratory procedures utilized in the present invention, in some embodiments, include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds.) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Strategies for Protein Purification and Characterization - A Laboratory Course Manual" CSHL Press (1996). Other general references are provided throughout this document.

### Materials and methods

### Cellular transduction

L6 myoblasts were transduced using a pQCXIP retroviral plasmid, and then underwent positive selection with 10µg/ml of Blasticidin HCl (Clbiochem), as previously described (Niu et al., (2010); Am. J. Physiol. Endocrinol. Metab.).

### Scaffold Fabrication

Porous biodegradable scaffolds were made using salt leaching technique to achieve pore sizes of 212-600 µm, out of poly-l-lactic acid (PLLA;Polysciences, Warrington) and polylactic glycolic acid (PLGA;Boehringer Ingelheim). A 5% (w/v) polymer solution of each polymer was prepared separately, by dissolving 0.5 gr polymer in 10 ml chloroform, in a small glass vial which was placed on a magnetic stirring plate overnight (ON). The polymers were then mixed together in a 1:1 ratio, to create a PLLA/PLGA solution. NaCl was sieved to 212-600 µm particles and 0.4 gr was poured into Teflon cylinder molds (18 mm internal diameter) and dissolved in 0.24 ml PLLA/PLGA solution. The Teflon molds were left ON to allow for chloroform evaporation, after which, the scaffolds were gently removed from the molds and placed into histology cassettes. The salt was leached by placing the histology cassettes in a beaker filled with distilled water, on a magnetic stirring plate; the water was exchanged every hour for 6-8 hours. The scaffolds were then removed from the histology cassettes, dried on a Kimwipe and frozen ON at - 80 °C, in a 50-ml tube. Finally, the scaffolds were lyophilized ON and kept dry under vacuum until use. One day prior to seeding, round pieces of 6mm diameter were prepared using a biopsy punch (Miltex). These pieces were sterilized in 70% ethanol ON. Before seeding, the scaffolds were washed twice in phosphate buffer saline (PBS) (Sigma) and dried using vacuum.

### Cell Culture

L6WT and L6GLUT4 myoblasts were cultured in MEM Alpha medium (Biological Industries) supplemented with 10% of fetal bovine serum (FBS) (Hyclone), 1% Penstrep antibiotic (PS) (Biological Industries) and 0.1% Amphotericin B (Amp B) (antifungal) (Biological Industries). Cells are incubated in a 5% CO₂ humidified atmosphere at 37 °C. L6GLUT4, were added directly in the culture plate with 2 µg/ml of Blasticidin HCl (Clbiochem), antibiotic for positive selection of GLUT4 over-expressing cells.

### Three-dimensional tissue construction

Myoblasts were cultured up to 70% confluency and then washed with PBS and trypsionized with 2X Trypsin-EDTA (Biological Industries), centrifuged at room temperature (RT) at 0.2 rcf for 4 minutes and re-suspended in 6µl of a 1:1 mixture of 15 mg/ml fibrinogen (Sigma) and a 10-20 U/ml thrombin (Sigma). The cell suspension, 0.25/0.5 × 10⁶ cells per scaffold, was seeded into PLLA/PLGA scaffolds and allowed to dry for 30 minutes at 37 °C and 5% CO₂, inside a 12-well plate. After solidification, 2 ml of growth medium (MEM Alpha, 10% FBS, 1% PS and 0.1% Amp B) were added to each well. After 2 days, the scaffolds were transferred to a new 12-well plate using a sterile forceps and 2 ml of differentiation medium (MEM Alpha, 2% FBS, 1% PS and 0.1% Amp B) were added to each well. For scaffolds seeded with L6GLUT4 cells, each well may optionally be supplemented with 2 µg/ml of Blasticidin HCl.

### Whole-mount scaffolds staining

Scaffolds seeded with L6WT of L6GLU4 were cultured for 1 week, 2 weeks and 3 weeks *in-vitro.* Whole scaffolds were washed with PBS solution and then fixated in 4% paraformaldehyde for 20 min, the fixation solution was removed, and the scaffolds were washed with PBS. Next, the scaffolds were treated with 0.3% Triton X-100 for 10 min, in order to permeabilize the cells. Then the scaffolds were washed with PBS and soaked in blocking solution (10% FBS (v/v), 0.1% Triton (v/v), 1% Glycine (w/v)) ON at 4 °C. The following day samples were incubated ON at 4 °C, with a solution of primary antibodies diluted in blocking solution. Lastly, scaffolds were washed with PBS and incubated with secondary antibodies solution diluted in PBS for 3 hours at room temperature. Next, scaffolds were washed and stored in PBS until imaging.

### Glucose uptake evaluation by the three-dimensional constructs

L6WT and L6GLUT4 cells were seeded on PLLA/PLGA scaffolds, at a seeding concentration of 0.25/0.5 × 10⁶ cells/scaffold, in a 12 well-plate, and cultured for up to 3 weeks *in-vitro.* After 3 weeks, the constructs were washed with PBS and then were serum-starved for 3 hours, followed by 30 minutes incubation in either absence or presence of 100 nM insulin (Basal and Insulin, respectively). 2-[³H]-deoxyglucose (2DOG; 0.1mM; 2 µCi) was then added for additional 20 minutes. The uptake was then terminated by three rapid washes in ice-cold Krebs-Ringer-Phosphate (KRP) buffer. Following the last wash, the scaffolds were minced manually with fine scissors, vortexed and transferred to 500 µl of 1% SDS/1N NaOH solution at 85 °C for 1 hour, 50 µl samples were taken to measure cellular protein levels by BCA protein assay. The rest was added to 5 ml scintillation fluid in plastic minivials. 2DOG-associated radioactivity was counted using a beta-counter.

### BCA assay

Protein levels for each construct were determined using BCA kit (Thermo Scientific). In a 96-well plate, the standards were prepared, in duplicates, out of a stock of 2 µg/µl of bovine serum albumin (BSA). Each construct sample that was 7.5 µl in duplicates, were added to 30 µl of 1%SDS/1N NaOH solution. Then, 150 µl of a mix of reagent A and reagent B (comprising 15 ml of reagent A and 300 µl of reagent B) were added to each well. The plate was incubated at 37 °C for 30 minutes and cooled at RT for 10 minutes. Afterwards the optical density (OD) was read at a wave length of 562 nm using a plate reader.

### Implant Procedure - Intra-muscular implantation in the Rectus Abdominis muscle

The engineered construct was implanted after an *in-vitro* incubation of 2-3 weeks. Animals were anesthetized using a ketamine-xylazine cocktail (100 mg/Kg body weight (BW) and 10 mg/Kg BW, respectively) delivered with a 27-gauge needle via an i.p (intra-peritoneal) injection. A portion of the fur of the animal was removed by shaving and applying depilation cream, at the abdomen. A small incision in the skin was made allowing access to the linea-alba and surrounding tissue, where approximately 6 mm diameter defect was created by removing a full thickness tissue section from the abdominal wall. The constructs were sutured in place with four 8-0 silk sutures. Outer skin was closed and sutured using 5-0 AssuCryl surgical sutures. Animals were monitored closely for 1-2 hours to ensure full recovery.

### Glucose Tolerance Test - GTT assay

Glucose basal level was established using a glucometer, by a blood test following 5-6 hour or overnight fasting of the animals. Then, the animals were injected i.p with a 10% (w/v) glucose solution. Blood samples were obtained at 15, 30, 45, 60, 75, 90 and 120 minutes following glucose administration.

### EXAMPLE 1

### Morphology and differentiation of rat skeletal muscle cells

Initially the myoblasts (skeletal muscle cells) were characterized for muscle morphology and ability to differentiate and form muscle fibers on the PLLA/PLGA scaffolds. L6WT and L6GLUT4 over-expressing cells were seeded at a concentration of 0.5 × 10⁶ cells/scaffold, and grown for 1, 2 and 3 weeks *in-vitro.* Following the incubation period, whole scaffolds were fixated and stained for different myogenic markers and the presence of the glucose transporter GLUT4; Desmin: a muscle-specific type III intermediate filament; Myosin heavy chain (MYH): a motor protein of muscle thick filaments; and Myogenin (MYOG):a muscle-specific transcription factor involved in the coordination of skeletal muscle development, differentiation and repair.

The inventors demonstrated that as early as 1-week post seeding, L6 cells expressed the different myogenic markers, meaning the cells undergone differentiation and formed fibers on PLLA/PLGA scaffolds (Figs. 2 and 3). A greater GLUT4 expression signal was observed for the over-expressing cells (Fig. 2). After as early as 1 week of culturing, GLUT4 over-expressing cells were found to be positive for the muscular differentiation marker MYOG.

Cell differentiation was also analyzed after prolonged *in-vitro* culturing of 2 and 3 weeks. High expression of desmin, and the formation of defined and aligned muscle fibers throughout the whole scaffold, was observed for both WT and GLUT4 over-expressing cells (**Fig. 4**).

### EXAMPLE 2

### Glucose uptake analysis in vitro

To evaluate the glucose uptake ability of the three-dimensional constructs, a 2-[³H]-deoxyglucose (2DOG) uptake assay was performed. L6WT and L6GLUT4 myoblasts were seeded at a concentration of 0.25 or 0.5 × 10⁶ cells/well in 12 well-plate and cultured for 1, 2 or 3 weeks. After the *in-vitro* culturing periods, scaffolds were incubated in either the absence or presence of insulin (Basal and Insulin levels, respectively), and afterwards 2DOG was added to the samples. 2DOG is an analog of glucose and is radioactively labeled with tritium (³H). The 2DOG has the 2-hydroxyl group replaced with hydrogen, so it cannot undergo further glycolysis, and thus its uptake can be quantified. 2DOG associated radioactivity was counted using a beta-counter.

Upon seeding of 0.25 × 10⁶ cells per scaffold, the stimulated uptake by the L6GLUT4 cells was significantly higher compared to the basal uptake rate for L6GLUT4. No significant difference between the insulin stimulated uptake rates for the L6WT and L6GLUT4 constructs was observed (**Fig. 5**). However, upon increase of the cell concentration to 0.5 × 10⁶, a significantly higher insulin stimulated uptake rate was noted in the L6GLUT4 samples compared to L6WT (**Fig. 6**).

Next, the inventors wanted to determine whether higher cell concentration, will show higher glucose uptake for a shorter culturing period. Indeed, 3 weeks of culturing were shown to be preferable in terms of 2DOG uptake (**Fig. 7**). The inventors also noted that the uptake rate for 2 weeks was lower that for 1 week.

### EXAMPLE 3

### Glucose uptake in vivo

The animals used for the *in-vivo* experiments were RAG/MKR mice. These animals are immuno-deficient, having no mature B and T lymphocytes (RAG), and a genetically defective insulin receptor (MKR) leading to insulin-resistance and DM2 phenotype. Only males present the diabetic phenotype at the age of 8 weeks, and thus were implanted with a 6-mm scaffold, seeded with L6GLUT4 cells, in their abdominal muscle. In order to test the efficacy of the implants, a glucose tolerance test (GTT) was performed. The mice were injected i.p (intraperitoneal, injection into the body cavity) with a high dose (10% [w/v]) of glucose. It was shown by the inventors that due to their insulin resistance state, RAG/MKR mice, as oppose to their genetic background WT-FVBN counterparts, had a decreased tolerance for glucose and were unable to return to their basal glucose level (**Fig. 8**).

Eighteen (18) males at the age of 9-10 weeks were separated into 3 groups: 8 mice received constructs seeded with GLUT4 overexpressing L6 cells (OEG4 EMC), for control 6 mice did not receive any implant and 4 mice received empty scaffolds seeded with no cells and embedded with fibrin, to ensure that the implantation procedure does not affect the glycemic profile. The inventors demonstrated that mice which were implanted with GLUT4, showed better tolerance to glucose load compared to control mice, and their blood glucose levels returned to the basal - normal level faster compared to the control group (**Fig. 9**).

These data demonstrated that the *in-vitro* three-dimensional constructs of L6GLUT4 over-expressing cells, had higher glucose uptake ability compared to WT control. The inventors demonstrated using these *in-vivo* results, that overexpression of the GLUT4 transporter enhances overall increase in whole body glucose uptake and significantly improves blood glucose levels in DM2 a mammalian model organism.

### EXAMPLE 4

### GLUT4-overexpressing satellite cells

Stalitte cells (SC) are skeletal muscle precursor cells that are located beneath the basal lamina and comprise up to 1-5% of total skeletal muscle. They remain in quiescent state and can proliferate and undergo further differentiation following injury.

SCs were isolated from the tibialis muscle from the hind limbs of 8 week old C57BL6 mice. The hind limb was sterilized with 70% (v/v) ethanol and a cut was made at the ankle in order to expose the underline tissue. The outer skin and fascia were removed. The muscle was separated from the bone and cartilage and washed 3 times with cold DMEM supplemented with 1% PS. Next the muscle was dissected and minced, and the cut segments were placed under agitation for 1 hour at 37 °C in 0.25% trypsin-EDTA solution for enzymatic dissociation and then filtered through 100 µm membrane. The filtrate was centrifuged twice at room temperature (RT) for 10 min at 1,300 g. The pellet was re-suspended with BIO-AMF-2 medium and plated on a 0.1% gelatin coated plate and incubated overnight (O.N.) in a 5% CO₂ humidified atmosphere at 37 °C. Forty eight (48) hours after harvest, myogenic cells were separated from fibroblasts using a plating technique based on their adherence to gelatin-coated surfaces. Cells were treated with 2× trypsin-EDTA, and centrifuged at RT for 10 minutes at 1,300 g. The pellet was re-suspended in DMEM supplemented with 10% FBS and 1% PS, and plated on a non-gelatin coated plate and incubated for 2 hours. This step was repeated twice. The fibroblasts adhered to the surface of the plate and the SC were suspended in the medium in the plate, which was collected from each incubation, centrifuged, re-suspended in BIOAMF-2 medium and plated on a gelatin coated plate.

### Morphological characterization of mouse isolated SC

The inventors sought for suitble differentiation medium for the SC. For this SC were seeded at a concentration of 50,000 cells\well in a 24 well-plate. The cells were cultured *in-vitro* for a week, in 4 different mediums:
- BIO-AMF-2, SC growth medium
- DMEM medium, supplemented with 5% horse serum (HS) and 1% penstrep
- DMEM medium, supplemented with 2% FBS and 1% penstrep
- SKMCM medium, skeletal muscle cells growth medium, supllemented with 2% FBS and 1% penstrep

The SC has a single cell morpholgy (circle in **Fig. 10A**) and under high confluency or in the precense of differentiation factors it can differentiate into a myocyte (squre in **Fig. 10A**). The inventors noted that the SC had the best differentiation rate with the DMEM supllemented with HS (**Fig. 11B**), as defined by the presence of multinucliated fibers. The differentiated C57-SC cells formed defined multinuclated elongated fibers **(****Fig. 11****).**

### Chahracterization of C57-SC engineered contructs

Isolated C57-SC were seeded at a concentration of 0.5 × 10⁶ and 1 × 10⁶ on PLLA\PLGA scaffolds and cultured *in-vitro* for 3 weeks in the chosen differentiation medium (DMEM supllemented with HS). After the culturing period the constructs were fixated and stained for desmin and MYOG, to assess the formation of muscle tissue.

Differentiation and fiber formation were observed in both of the tested cell concentrations. The fibers for the higher initial concentration were more defined throughout the scaffold (**Figs.12** **and** **14**). However, quatification of the desmin signal showed no significat difference between the two concentrations (**Fig. 13**).

### GLUT4 Transduction

In order to establish stable over-expressing GLUT4 cells, C57-SCs were transduced with pQCXIB retroviral plasmid. The gene for GLUT4 was on the pQCXIB retroviral plasmid and the GP2-293 packing cells for viral production were used. The plasmid had the selection marker for Blasticidin antibiotic.

A day prior to the transfection, GP2-293 cells were seeded at a concentration of 4 × 10⁶ in a 10 cm plate. The following day 15 µg of the target plasmid, 15 µg of envelope plasmid and 9 µl of Xfext reagent were added to the packing cells to produce the viral particles. After 72 hours, retroviral supernatant was harvested and filtered through a 0.45 µm filter to remove cellular debris. The viral filtrate was diluted to the desired concentration and added to the cells, that were seeded a day before, for the transduction. The following day the viral particles were removed, and the cells were allowed to expand for 48 hours. Next, the inventors positively selected cells with 10 µg/ml of Blasticidin HCl antibiotic, according to the manufacturer's instructions (Clontech Laboratories, Inc., manual PT3132-1 (061113), Cat. No. 631530).

To further optimize transduction conditions which will provide higher yield of GLUT4 over-expressing cells, a large calibration assay was preformed, as follows.

The inventors tried 2 different envelope plasmids for the packing, 3 different virus concentrations, and 2 different facilitating agents, each at 4 concentrations. The conditions are detailed in table 1.

**Table 1: Transduction calibration conditions**

| Envelope plasmid: | | | | | | | | |
|---|---|---|---|---|---|---|---|---|
| | p4070A (Ampho) | | | | pgap70 (Eco) | | | |

| Virus concentration (% of total medium volume): | | | | | | | | |
|---|---|---|---|---|---|---|---|---|
| | | | 25 | | 33 | | 50 | |

| Facilitating agent: | | | | | | | | |
|---|---|---|---|---|---|---|---|---|
| | Polybrene (µg/ml) (PB) | | | | Retronectin (µg/ml) (RN) | | | |
| | 2 | 4 | 6 | 8 | 20 | 50 | 75 | 100 |

The resulting clones were analyzed to determine the over-expression, using immunofluorescent staining for GLUT4 transporter.

The description of the the first batch of clones which was analyzed, is presented in table 2. **Figure 15** shows the staining of the GLUT4 transpoeter in these clones (table 2) compared to the WT cells (isolated and not transduced). **Figure 16** is a bar graph showing the quntification of the signal intensity of the immunofluorescent micrographs of **Fig. 15****.**

**Table 2: Description of the first batch of clones**

| | |
|---|---|
| C57WT | PB A12 |
| | 2 µg/ml ; 25% |
| RN A12 | PB A34 |
| 20 µg/ml ; 25% | 2 µg/ml ; 33% |
| RN A34 | PB A56 |
| 20 µg/ml ; 33% | 2 µg/ml ; 50% |
| RN A56 | PB B12 |
| 20 µg/ml ; 50% | 4 µg/ml ; 25% |

The description of the the first batch of clones which was analyzed, is presented in table 3. **Figure 17** shows the staining of the GLUT4 transpoeter in these clones (table 3) compared to the WT cells (isolated and not transduced). **Figure 18** is a bar graph showing the quantification of the signal intensity of the immunofluorescent micrographs of **Fig. 18****.**

**Table 3: Description of the second batch of clones**

| | |
|---|---|
| C57WT | PB B34 |
| | 4 µg/ml ; 33% |
| RN B12 | PB B56 |
| 50 µg/ml ; 25% | 4 µg/ml ; 50% |
| RN B34 | PB C12 |
| 50 µg/ml ; 33% | 6 µg/ml ; 25% |
| RN B56 | RN C12 |
| 50 µg/ml ; 50% | 75 µg/ml ; 25% |

Fluorescent images and the quntification of the signals **(****Figs. 15-18****),** showed that the inventors had succsesfuly transduced naïve cells to over-express GLUT4.

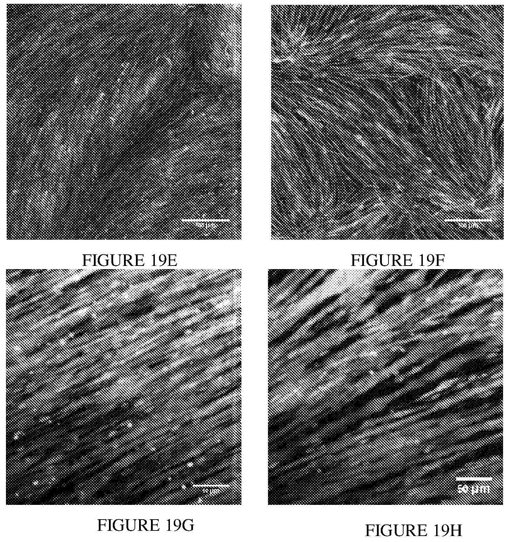

Clones PB-A12 and RN-B12 were then seeded in an opticic 24 well-plate at a concentration of 50,000 cells\well and cultured for 1 week in-vitro. The formed myotubes were stained for the presence of the myogenic markers: desmin, MYH and MYOG. **Fig. 19** shows defined myotubes exprssing all of the tested markers.

The next step was to assess the ability of the transduced cells to uptake glucose. In order to calibrate the cell number required to preform a 2DOG uptake assay, asmentioned above, the inventors seeded: WT C57-SC, and the RN-A56 and PB-A34 clones, in a 12 well plate, in different concentrations (50,000, 100,00, 175,000 and 250,000 cells per well), cultured, and followed the cells for up to 10 days.

**Figs. 20-22** show that the transduced cells retained fusion ability and form defined myotubes. While full differentiation was observed for the 100,000 cells/well, for 2DOG uptake assay the inventors chose the 250,000 cells/well and 1 week of culturing, as these were the conditions for the L6 cells, mentioned hereinabove.

WT C57-SC and the clones PB-A12 and PBA34, were seeded at a concentration of 250,000 cells/well and cultured for 1 week, afterward a 2DOG uptake assay was performed. The uptake ability of 2 clones of the transduced cells compared to the WT cells is shown **(****Fig. 23****).** Both clones exhibited significantly higher basal and insulin stimulated glucose uptake. Clone PB-A12 demonstrated a higher glucose uptake rate than the PB-A34 rate.

The results show that it is feasbile to isolate myogenic progenitor cells from a muscle biopsy, and further transduce these cells to overexpress GLUT4, which have relevant clinical usage for glycemic regulation.

### EXAMPLE 5

### GLUT4-overexpressing SCs regulate glycemic homeostasis in vivo

SCs isolated and transduced to overexpress GLUT4, as mentioned hereinabove e.g., as shown in **Figs. 16** **and** **18****,** are analyzed for glucose uptake ability. The best preforming clones, are then in vivo implanted (for example in DIO model C57BL6 mice). Glycemic values are recorded both under fasting conditions and postprandial, according to methods as described hereinabove (e.g., GTT). A SC clone which maintaines a glucose homeostasis at levels of: less than or equal to 120 milligrams/Deciliter (mg/dL) at fasting; and less than 160 mg/dL postprandial, can potentially be used as a therapeutic agent for regulating glycemic homeostasis in vivo.

While the present invention has been particularly described, persons skilled in the art will appreciate that many variations and modifications can be made. Therefore, the invention is not to be construed as restricted to the particularly described embodiments, and the scope and concept of the invention will be more readily understood by reference to the claims which follow.

## Claims

1. A scaffold-cell construct comprising:
a. a porous scaffold comprising at least one biocompatible polymer comprising a combination of poly-1-lactic acid (PLLA) and polylactic glycolic acid (PLGA), or collagen; and
b. a cell population deposited on or in said scaffold comprising recombinant myoblasts: (i) comprising an exogenous polynucleotide encoding glucose transporter type 4 (GLUT4); (ii) over-expressing said GLUT4; and (iii) having increased GLUT4 activity, compared to control wild type myoblast cells,
and wherein said scaffold-cell construct is transplantable.

2. The scaffold-cell construct of claim 1, being **characterized by** having greater glucose uptake rates compared to said control wild type myoblast cells.

3. The scaffold-cell construct of claim 1 or 2, wherein said PLLA and said PLGA are in a ratio of 3:1 - 1:3 w/w ratio.

4. The scaffold-cell construct of any one of claims 1-3, wherein said biocompatible polymer comprises interconnected pores, wherein at least 80% of said pores have a diameter of between 200 and 600 microns.

5. The scaffold-cell construct of any one of claims 1-4, for use in restoring glucose homeostasis levels in a subject in need thereof, wherein:
a. said glucose levels are:
i. less than or equal to 100 mg/dL at fasting; and
ii. lower than 140 mg/dL postprandial; or
b. said glucose levels are:
i. less than or equal to 120 mg/dL at fasting; and
ii. lower than 160 mg/dL postprandial.

6. The scaffold-cell construct of any one of claims 1-5, for use in the treatment or prevention of diabetes mellitus or metabolic syndrome in a subject in need thereof, preferably wherein said metabolic syndrome is selected from: obesity, pre-diabetes and insulin resistance or related to insulin resistance.

7. A composition comprising the scaffold-cell construct of any one of claims 1-4.

8. A method of making a scaffold-cell construct for restoring glucose homeostatic levels in a diabetic subject in need thereof,
the method comprising contacting recombinant myoblasts: (i) comprising an exogenous polynucleotide encoding GLUT4; (ii) over-expressing said GLUT4; and (iii) having increased GLUT4 activity, compared to control wild type myoblast cells, with a scaffold comprising at least one biocompatible polymer comprising a combination of PLLA and PLGA or collagen, and wherein said scaffold-cell construct is transplantable.

9. The method of claim 8, further comprising culturing said recombinant myoblasts on or in said scaffold for at least 7 days.

## Patentansprüche

1. Gerüstzellkonstrukt, umfassend:
a. ein poröses Gerüst, das mindestens ein biokompatibles Polymer umfasst, welches eine Kombination aus Poly-L-Milchsäure (PLLA) und Poly(milchsäureglykolsäure) (PLGA) oder Kollagen umfasst; und
b. eine auf oder in dem Gerüst abgelagerte Zellpopulation, die rekombinante Myoblasten umfasst: (i) umfassend ein exogenes Polynukleotid, das Glukosetransporter Typ 4 (GLUT4) codiert; (ii) Überexpression des GLUT4; und (iii) das erhöhte GLUT4-Aktivität im Vergleich zu Kontroll-Wildtyp-Myoblastenzellen aufweist,
und wobei das Gerüstzellkonstrukt transplantierbar ist.

2. Gerüstzellkonstrukt nach Anspruch 1 **dadurch gekennzeichnet, dass** es im Vergleich zu den Kontroll-Wildtyp-Myoblastenzellen eine höhere Glukoseaufnahmerate aufweist.

3. Gerüstzellenkonstrukt nach Anspruch 1 oder 2, wobei das PLLA und das PLGA in einem Verhältnis von 3:1 - 1:3 Gew.-Verhältnis vorhanden sind.

4. Gerüstzellkonstrukt nach einem der Ansprüche 1-3, wobei das biokompatible Polymer miteinander verbundene Poren umfasst, wobei mindestens 80% der Poren einen Durchmesser zwischen 200 und 600 Mikrometern aufweisen.

5. Gerüstzellkonstrukt nach einem der Ansprüche 1-4 zur Verwendung bei der Wiederherstellung des Glukose-Homeostasisniveaus bei einem Subjekt, der dies benötigt, wobei:
a. die Glukoseniveaus sind:
i. kleiner oder gleich 100 mg/dL beim Fasten; und
ii. niedriger als 140 mg/dL postprandial; oder
b. die Glukosniveaus sind:
i. kleiner oder gleich 120 mg/dL beim Fasten; und
ii. niedriger als 160 mg/dL postprandial.

6. Gerüstzellkonstrukt nach einem der Ansprüche 1-5, zur Verwendung bei der Behandlung oder Vorbeugung von Diabetes mellitus oder des metabolischen Syndroms bei einem Subjekt, der dies benötigt, wobei das metabolische Syndrom vorzugsweise ausgewählt ist aus: Fettleibigkeit, Prädiabetes und Insulinresistenz oder in Bezug auf Insulinresistenz.

7. Zusammensetzung, umfassend das Gerüstzellkonstrukt nach einem der Ansprüche 1-4.

8. Verfahren zur Herstellung eines Gerüstzellkonstrukts zur Wiederherstellung des Glukose-Homeostatisniveaus bei einem diabetischen Subjekt, der dies benötigt, wobei das Verfahren das Inkontaktbringen rekombinanter Myoblasten umfasst: (i) die ein exogenes Polynukleotid umfassen, das GLUT4 codiert; (ii) GLUT4 überexprimieren; und (iii) die im Vergleich zu Kontroll-Wildtyp-Myoblastenzellen eine erhöhte GLUT4-Aktivität aufweisen, mit einem Gerüst, das mindestens ein biokompatibles Polymer umfasst, das eine Kombination aus PLLA und PLGA oder Kollagen umfasst, und wobei das Gerüstzellkonstrukt transplantierbar ist.

9. Verfahren nach Anspruch 8, weiter umfassend Kultivieren der rekombinanten Myoblasten auf oder in dem Gerüst mindestens 7 Tage lang.

## Revendications

1. Construction de cellule d'échafaudage comprenant :
a. un échafaudage poreux comprenant au moins un polymère biocompatible comprenant une combinaison d'acide poly-1-lactique (PLLA) et d'acide polylactique glycolique (PLGA), ou de collagène ; et
b. une population de cellules déposée sur, ou dans, ledit échafaudage comprenant des myoblastes recombinants : (i) comprenant un polynucléotide exogène codant pour le transporteur de glucose de type 4 (GLUT4) ; (ii) surexprimant ledit GLUT4 ; et (iii) présentant une activité de GLUT4 accrue, par comparaison à des cellules myoblastiques de type sauvage témoins,
et dans laquelle ladite construction de cellule d'échafaudage est transplantable.

2. Construction de cellule d'échafaudage selon la revendication 1, qui est **caractérisée par** ce qu'elle présente des taux d'absorption de glucose plus important par comparaison à ceux desdites cellules myoblastiques de type sauvage témoins.

3. Construction de cellule d'échafaudage selon la revendication 1 ou 2, dans laquelle ledit PLLA et ledit PLGA sont dans un rapport de 3:1 - 1:3 p/p.

4. Construction de cellule d'échafaudage selon l'une quelconque des revendications 1-3, dans laquelle ledit polymère biocompatible comprend des pores interconnectés, dans laquelle au moins 80 % desdits pores présentent un diamètre compris entre 200 et 600 micromètres.

5. Construction de cellule d'échafaudage selon l'une quelconque des revendications 1-4, destinée à être utilisée dans la restauration de niveaux d'homéostasie du glucose chez un sujet qui en a besoin, dans laquelle :
a. lesdits niveaux de glucose sont :
i. inférieurs ou égaux à 100 mg/dl à jeun ; et
ii. inférieurs à 140 mg/dl postprandial ; ou
b. lesdits niveaux de glucose sont :
i. inférieurs ou égaux à 120 mg/dl à jeun ; et
ii. inférieurs à 160 mg/dl postprandial.

6. Construction de cellule d'échafaudage selon l'une quelconque des revendications 1-5, destinée à être utilisée dans le traitement ou la prévention du diabète sucré ou du syndrome métabolique chez un sujet qui en a besoin, de préférence dans laquelle ledit syndrome métabolique est choisi parmi : l'obésité, le prédiabète et la résistance à l'insuline ou lié à la résistance à l'insuline.

7. Composition comprenant la construction de cellule d'échafaudage selon l'une quelconque des revendications 1-4.

8. Procédé de fabrication d'une construction de cellule d'échafaudage pour restaurer les niveaux homéostatiques de glucose chez un sujet diabétique qui en a besoin, le procédé comprenant la mise en contact de myoblastes recombinants : (i) comprenant un polynucléotide exogène codant pour le GLUT4 ; (ii) surexprimant ledit GLUT4 ; et (iii) présentant une activité de GLUT4 accrue, par comparaison à des cellules myoblastiques de type sauvage témoins, avec un échafaudage comprenant au moins un polymère biocompatible comprenant une combinaison de PLLA et de PLGA ou de collagène, et dans lequel ladite construction de cellule d'échafaudage est transplantable.

9. Procédé selon la revendication 8, comprenant en outre la culture desdits myoblastes recombinants sur, ou dans, ledit échafaudage pendant au moins 7 jours.
